# EUROPEAN PATENT APPLICATION

(11) **EP 4 389 889 A2**
(43) Date of publication of application: **26.06.2024**
(21) Application number: 24170871.8
(22) Date of filing: 09.10.2018
(51) Int. Cl.: C12N 15/10

(54) **COMPOSITIONS AND METHODS FOR EDITING RNA**

(30) Priority: 06.10.2017 US 201762569376 P
(62) Divisional of application: 18865285.3
(71) Applicant: Oregon Health & Science University, Portland, OR 97239 (US)
(72) Inventor: Mandel, Gail, Portland, 97210 (US); Adelman, John P., Portland, 97239 (US); Sinnamon, John, Portland, 97239 (US)
(74) Representative: Nederlandsch Octrooibureau

(57) **Abstract**

Compositions and methods for editing endogenous RNA molecules are provided.

## Description

This application claims priority under 35 U.S.C. §119(e) to U.S. Provisional Patent Application No. 62/569,376, filed October 6, 2017. The foregoing application is incorporated by reference herein.

This invention was made with government support under Grant No. NS087726 awarded by the National Institutes of Health. The Government has certain rights in this invention.

### DESCRIPTION OF THE TEXT FILE SUBMITTED ELECTRONICALLY

The contents of the text file submitted electronically herewith are incorporated herein by reference in their entirety: A computer readable format copy of the Sequence Listing (filename: SEQLIST.txt; date recorded: October 9, 2018; file size: 44.6 KB).

### FIELD OF THE INVENTION

The present invention relates to the field of nucleic acid editing. Specifically, compositions and methods for therapeutically editing RNA, particularly endogenous RNA within the nucleus, are disclosed.

### BACKGROUND OF THE INVENTION

Several publications and patent documents are cited throughout the specification in order to describe the state of the art to which this invention pertains. Each of these citations is incorporated herein by reference as though set forth in full.

Advances have been made in strategies that edit or alter genetic material, e.g., various gene editing technologies. However, there remains a need for methodologies that correct disease-causing mutations, especially in the nervous system.

Rett syndrome is a neurodevelopmental disorder caused by sporadic mutations in the transcription factor Methyl CpG Binding Protein 2 (MECP2) (Amir, et al. (1999) Nat. Genet., 23:185-188). MECP2 is located on the X chromosome. Because of dosage compensation mechanisms in mammals, females affected with Rett syndrome are mosaic, with an approximately 50:50 split between wild-type and mutant cells. Females with MECP2 mutations undergo regression of early developmental milestones, such as speech and purposeful hand motions, and then acquire severe motor abnormalities, including respiration, and die on average by age 40 (Neul, et al., (2010) Ann. Neurol., 68:944-950; Percy, et al. (2010) Ann. Neurol., 68:951-955). Males with mutations in MECP2, with a single X chromosome, have an even more profound disease, usually succumbing before 2 years of age (Schule, et al. (2008) Clin. Genet., 74:116-126). There is no cure for Rett syndrome.

### SUMMARY OF THE INVENTION

In accordance with one aspect of the instant invention, methods for editing the sequence of an endogenous RNA in a cell, particularly in the nucleus of the cell, are provided. In a certain embodiment, the method comprises delivering to the cell i) a nucleic acid molecule encoding a fusion protein comprising a nuclear localization signal and an RNA editing enzyme linked to an RNA binding domain and ii) a nucleic acid molecule encoding one or more guide RNA. The guide RNA comprises a sequence specifically recognized by the RNA binding domain. The guide RNA also specifically hybridizes with a target sequence in the endogenous RNA and comprises a mismatch at a nucleotide to be edited. In certain embodiments, the RNA editing enzyme is an Adenosine Deaminase Acting on RNA (ADAR) such as ADRAR1 or ADAR2. In certain embodiments, the RNA binding domain is the λN peptide and the sequence specifically recognized by the RNA binding domain is the BoxB sequence. In certain embodiments, the endogenous RNA is methyl CpG binding protein 2 (MECP2) RNA. In certain embodiments, the nuclear localization signal is the SV40 Large T-antigen nuclear localization signal. The nucleic acid molecules of these methods may be contained within a single vector, such as a viral vector (e.g., adeno-associated virus (AAV) vector).

In accordance with another aspect of the instant invention, additional methods for editing the sequence of an endogenous RNA in a cell, particularly in the nucleus of the cell, are provided. In certain embodiments, the method comprises delivering to the cell a nucleic acid molecule encoding one or more guide RNA, wherein the guide RNA comprises a sequence(s) and/or structure specifically recognized by an endogenous deaminase such as human Adenosine Deaminase Acting on RNA (ADAR). The guide RNA also specifically hybridizes with the target sequence in the endogenous RNA and comprises a mismatch at a nucleotide to be edited. In certain embodiment, the ADAR is ADRA1 or ADAR2. In certain embodiments, the endogenous RNA is methyl CpG binding protein 2 (MECP2) RNA. In certain embodiments, the nucleic acid molecule is contained within a viral vector (e.g., an AAV vector).

In accordance with another aspect of the instant invention, additional methods for editing the sequence of an endogenous RNA in a cell, particularly in the nucleus of the cell, are provided. In certain embodiments, the method comprises delivering to the cell a nucleic acid molecule encoding a guide RNA (e.g., within an AAV), wherein the guide RNA comprises a sequence(s) and/or structure specifically recognized by an endogenous human Adenosine Deaminase Acting on RNA (ADAR). Accordingly, in various aspects, the present methods for editing can be undertaken in the absence of a recombinant RNA editing enzyme (e.g., in the absence of a recombinant ADAR). As such, in embodiments, the present methods engage endogenous ADAR activities to affect the editing described herein.

In accordance with another aspect of the instant invention, methods of treating, inhibiting, and/or preventing Rett syndrome in a subject are provided. In certain embodiments, the method comprises using the RNA editing methods of the instant invention. For example, the method may comprise administering to the subject a nucleic acid molecule encoding a fusion protein comprising an RNA editing enzyme linked to an RNA binding domain and a nucleic acid molecule encoding a guide RNA, wherein the fusion protein comprises a nuclear localization signal. In certain embodiments, the methods comprise administering to the subject a nucleic acid molecule encoding a guide RNA, wherein the guide RNA comprises a sequence(s) and/or structure(s) specifically recognized by an endogenous human deaminase such as Adenosine Deaminase Acting on RNA (ADAR), and wherein the guide RNA specifically hybridizes with methyl CpG binding protein 2 (MECP2) RNA and comprises a mismatch at the mutant nucleotide of the endogenous *MECP2* RNA.

### BRIEF DESCRIPTION OF THE DRAWINGS

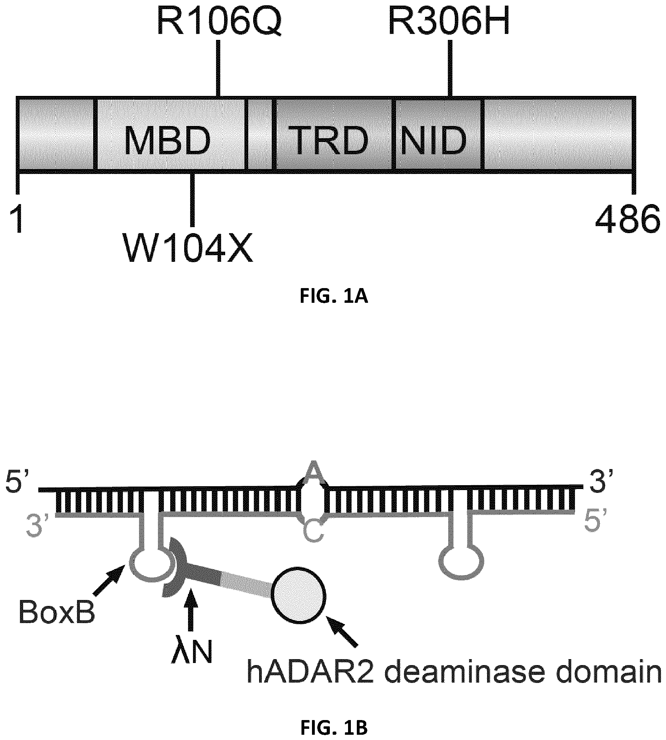

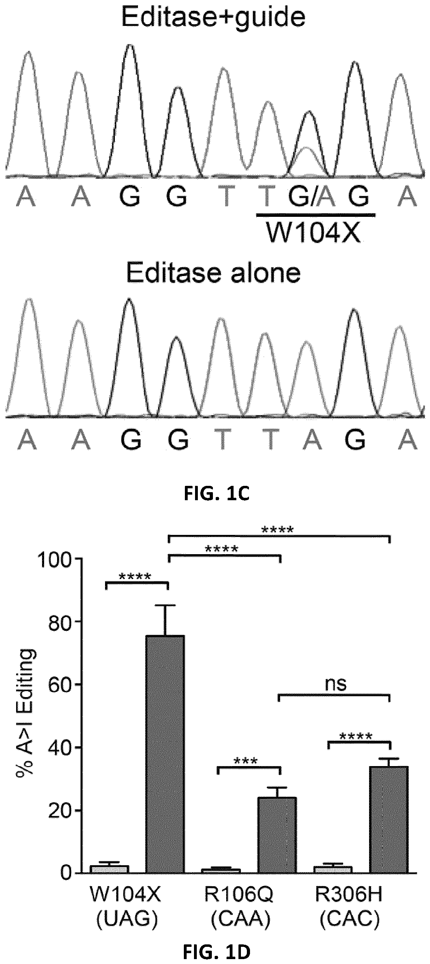

Figures 1A-1D show that editing efficiency is sequence-dependent. Fig. 1A: Schematic showing positions of three G > A mutations relative to the Methyl DNA Binding Domain (MBD), Transcriptional Repressor Domain (TRD), and NCoR interaction domain (NID) in MeCP2. Fig. 1B: Schematic of the core components of site-directed RNA editing. Hybrid Editase contains an RNA binding domain from bacteriophage λ (λN) and the catalytic domain (deaminase domain) of human Adenosine Deaminase Acting on RNA 2 (hADAR2). Also included, but not shown, are three copies of a nuclear localization signal (NLS) and two copies of a human influenza hemagglutinin (HA)-epitope tag. The guide RNA is complementary to *Mecp2* mRNA and contains the hairpin (stem loop) recognized by the λN RNA binding domain. In opposition to the target A, a C has been introduced into the guide to increase editing efficiency. Fig. 1C: Sequencing chromatograms of *Mecp2^{W104X}* cDNA after transfection into N2A neuroblastoma cells of Editase with (Top) or without (Bottom) guide. Fig. 1D: Percent A-to-I editing (mean ± SD; n = 3) quantified using direct sequencing of *Mecp2* cDNA and including data in Fig. 1C. Light-gray bars, cells transfected with Editase alone; dark-gray bars, cells transfected with Editase and guide. ****P* < 0.001, *****P* < 0.0001 by one-way ANOVA with Bonferroni post hoc test. ns: not significant.

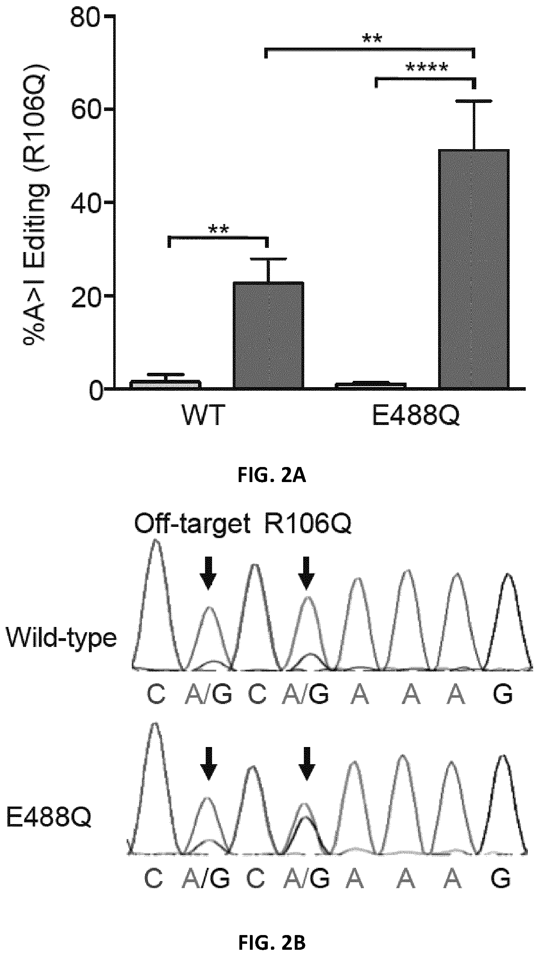

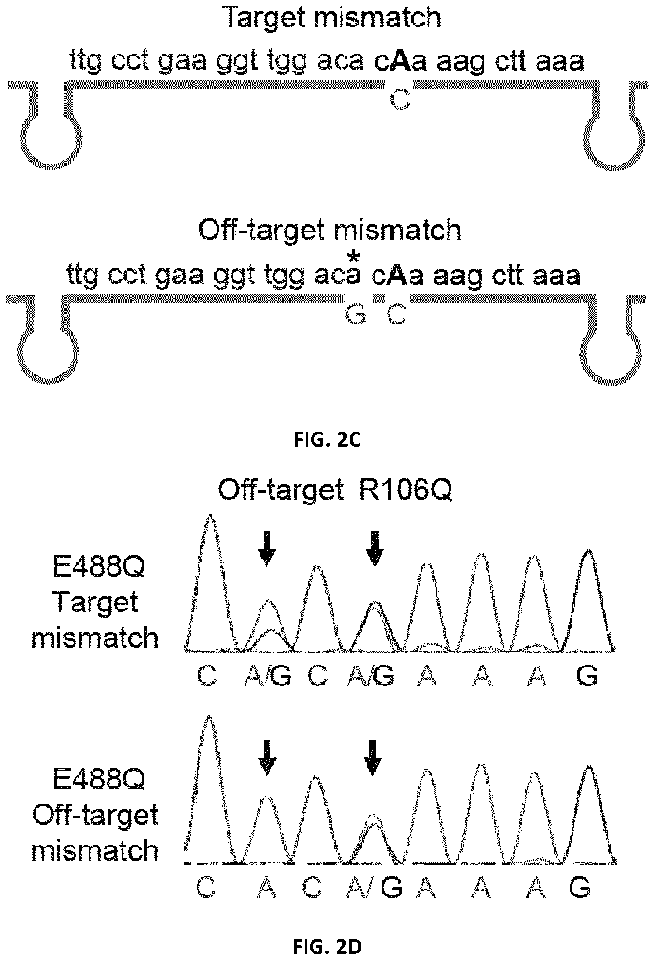

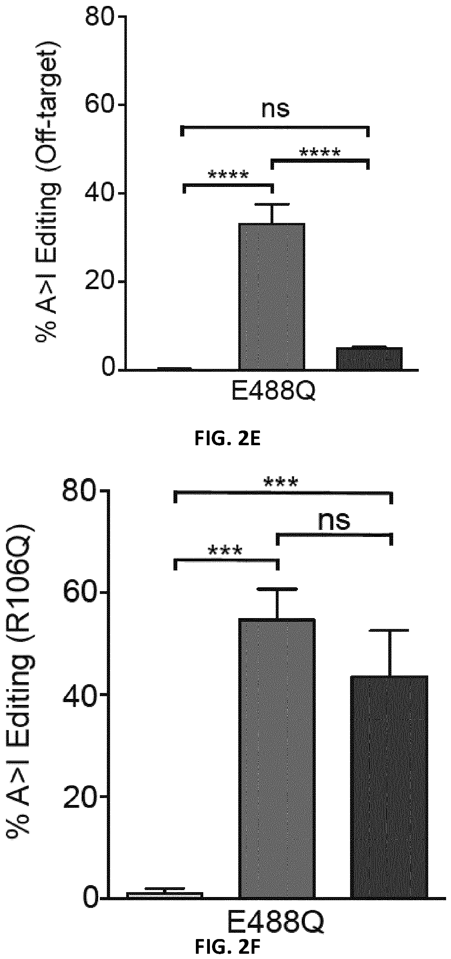

Figures 2A-2F show that off-target editing with a more efficient Editase can be reduced using a guide with a site-specific A-G mismatch to *Mecp2* mRNA. Fig. 2A: Percent A-to-I editing at the R106Q (mean ± SD, n = 3) site after transfection into N2A cells of guide RNA and Editase^{WT} or Editase^{E488Q} (mean ± SD; n = 3, includes data in Fig. 2B). Fig. 2B: Representative chromatograms of *Mecp2^{R106Q}* cDNA edited with Editase^{WT} (Top) or Editase^{E488Q} (Bottom). Fig. 2C: *Mecp2* mRNA relative to two different guide RNAs. The standard guide (Top) contains an A-C mismatch (R106Q) at the target A (highlighted in bold) to enhance editing. The modified guide (Bottom) contains an A-G mismatch at an off-target A marked by an asterisk to inhibit editing at this site. The provided target sequence is SEQ ID NO: 52. Fig. 2D: Chromatograms of *Mecp2* cDNA after transfection of N2A cells with Editase^{E488Q} and a guide containing only the mismatch at the target site (Top) or the modified guide containing both the on-target A-C mismatch and the A-G mismatch at the off-target site (Bottom). Fig. 2E: Off-target editing is severely reduced with the guide containing the A-G mismatch (mean ± SD; n = 3, includes data in Fig. 2D). Fig. 2F: Presence of the off-target A-G mismatch does not affect editing at the R106Q site (mean ± SD; n = 3, includes data in Fig. 2D). Light-gray bars, cells transfected with Editase alone; dark-gray bars, cells transfected with Editase and guide; black bars, cells transfected with Editase and guide containing the A-G mismatch. ***P* < 0.01, ****P* < 0.001, *****P* < 0.0001 by one-way ANOVA with Bonferroni post hoc test. ns: not significant.

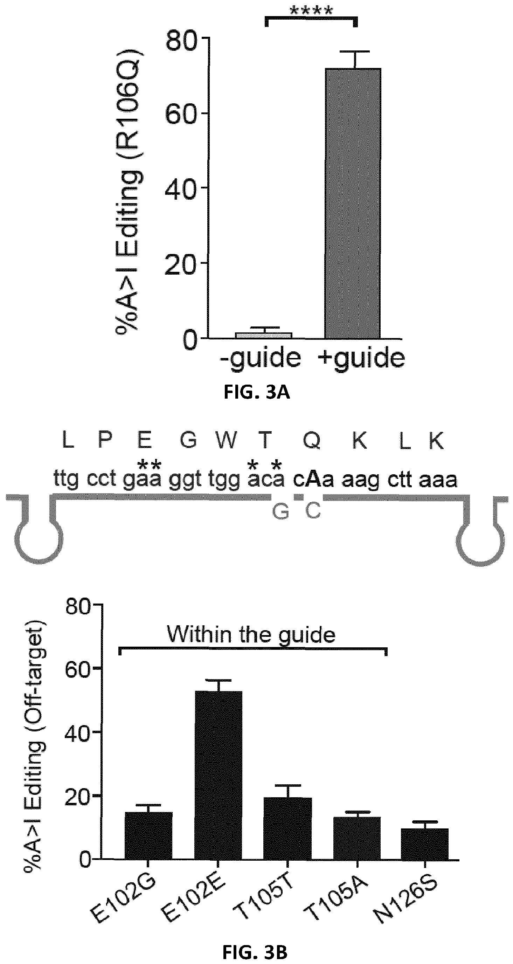

Figures 3A-3B shows sequence analysis of endogenous *MeCP2* mRNA following AAV1/2 transduction of primary neurons. Fig. 3A: Quantification of editing (mean ± SD, n = 3) by sequence analysis of cDNA isolated from *Mecp2*^{*R106Q*/*y*} hippocampal neurons (DIV14), 7 days following transduction with AAV1/2 virus. +guide refers to AAV1/2 that contains Editase under control of the neuronal *Synapsin* 1 promoter and six copies of the guide, each expressed under control of a U6 promoter. The guide contains a C mismatch at the targeted A for R106Q and a G mismatch at the off-target A T105T. The control virus encodes Editase under control of the *Synapsin I* promoter but lacks any guide sequences (-guide). *****P* < 0.0001 by unpaired two-tailed t test. Fig. 3B: *Mecp2* mRNA (SEQ ID NO: 52) and primary amino acid sequences (SEQ ID NO: 53) relative to the guide RNA region. The target A is bolded, and asterisks indicate off-target edited A residues. The hairpins in the guide represent the positions of the BoxB sequences recognized by λN peptide. The graph provides the quantitation of editing at the off-target sites within *Mecp2* mRNA (mean ± SD; n = 3). Residue N126S lies outside the guide region.

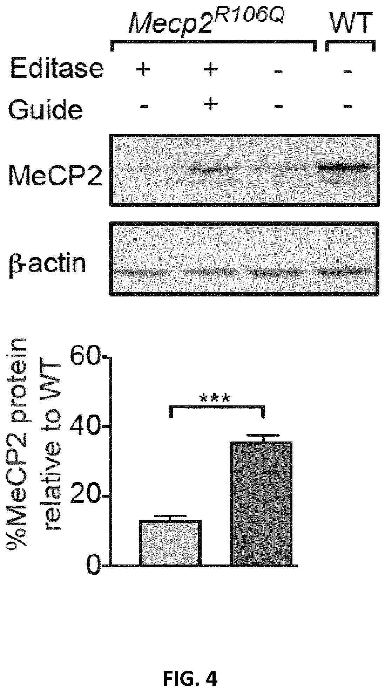

Figure 4 shows site-directed RNA editing increases MeCP2 protein levels, thereby demonstrating functional recovery of an endogenous disease causing protein after editing. A representative Western blot is provided of whole-cell lysates from *Mecp2*^{*R106Q*/*y*} or wildtype (WT, *Mecp2*^{*+*/*y*}) sibling hippocampal neurons (DIV14) transduced 7 days earlier with AAV1/2 expressing either Editase alone or Editase and guide. The guide contains a C mismatch at the R106Q site and a G mismatch at the off-target A, T105T. The graph provides a quantification of Western blots (mean ± SD, n = 3), each condition normalized to β-actin. Light-gray bar, cells transduced with Editase alone; dark-gray bar, cells transduced with Editase and guide. ****P* < 0.001 by unpaired two-tailed t test.

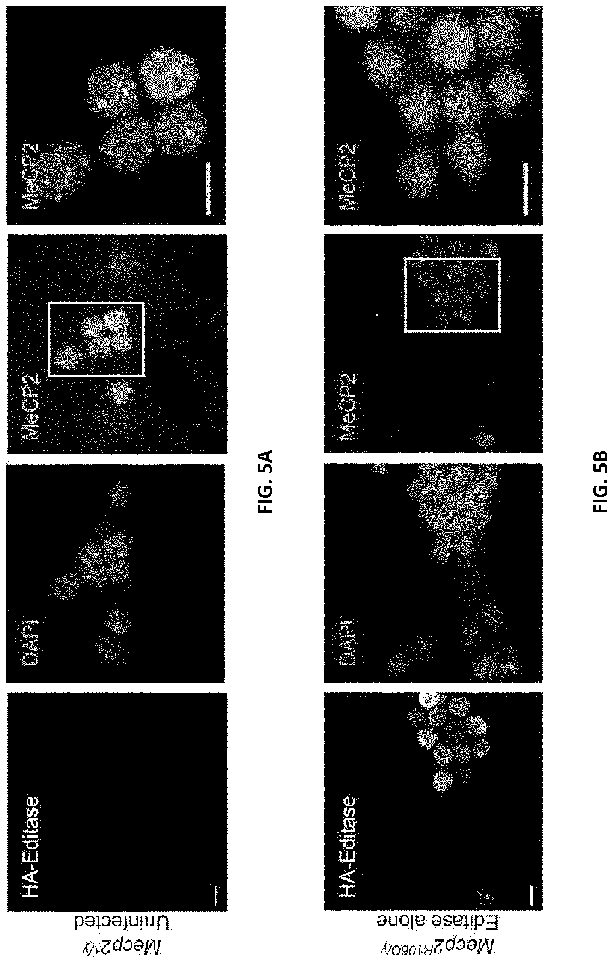

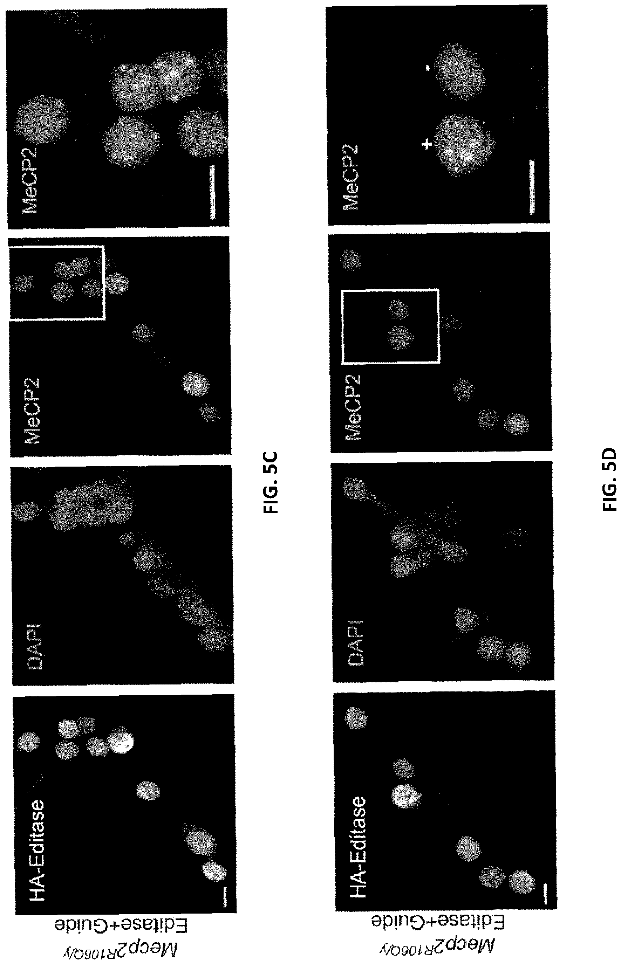

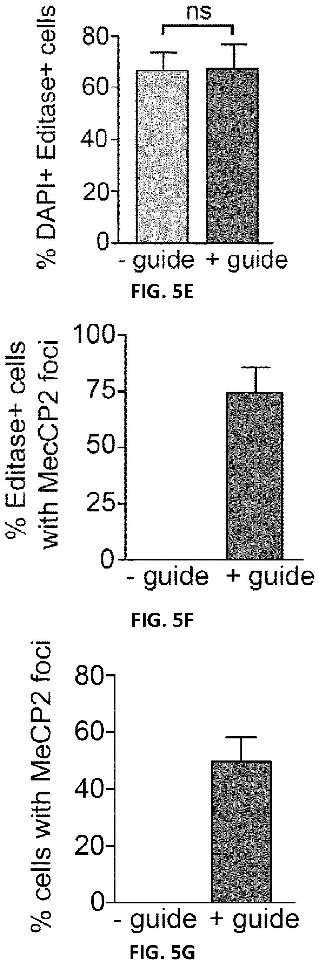

Figures 5A-5G show that site-directed RNA editing restores the ability of MeCP2 to bind to heterochromatin, demonstrating a recovery of function of an endogenous protein after editing. Shown are representative confocal images of hippocampal neurons (DIV14) immunolabeled for Editase (HA) and MeCP2. DAPI staining outlines the nuclei and shows heterochromatic foci. Insets demarcate the cells imaged at higher magnification and higher gain in the adjacent panels. Fig. 5A: Wild-type (*Mecp2*^{+/}*^{y})* neuronal cultures. Fig. 5B: *Mecp2*^{*R106Q*/*y*} neuronal cultures transduced with AAV1/2 virus expressing Editase alone (no guide). These neurons never exhibited MeCP2 enrichment in heterochromatin. Figs. 5C and 5D: *Mecp2*^{*R106Q*/*y*} neuronal cultures transduced with AAV1/2 virus expressing Editase and guide containing the C mismatch at the target A. In Fig. 5D, + and - indicate nuclei with the presence and absence, respectively, of MeCP2 enrichment in heterochromatin. Scale bar, 10 µm. Figs. 5E-5G: Each histogram represents quantification of cells (Editase alone, n = 134; Editase and guide, n = 137) from three fields in each of three slides (mean ± SD). Fig. 5E: Percentage of Editase+ cells identified by HA nuclear staining after thresholding signals from uninfected cells. Percentages are relative to the total number of DAPI+ cells. Fig. 5F: Percentage of Editase+ cells with MeCP2 enrichment in heterochromatin (foci). Fig. 5G: Percentage of all cells with MeCP2 enrichment in heterochromatin (foci). ns: not significant.

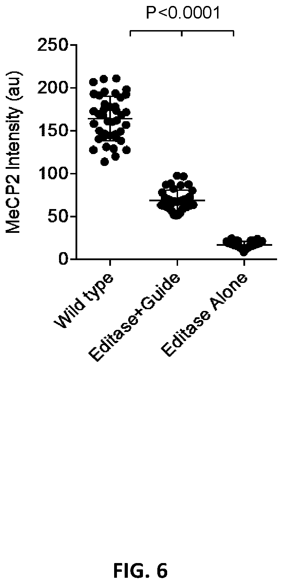

Figure 6 provides a graph of MeCP2 intensity in dentate neuronal heterochromatin in brains from wild-type mice or *Mecp2^{317G>A}* (*Mecp2^{R106Q}*) mice treated with AAV vectors encoding Editase alone or Editase with guide RNAs.

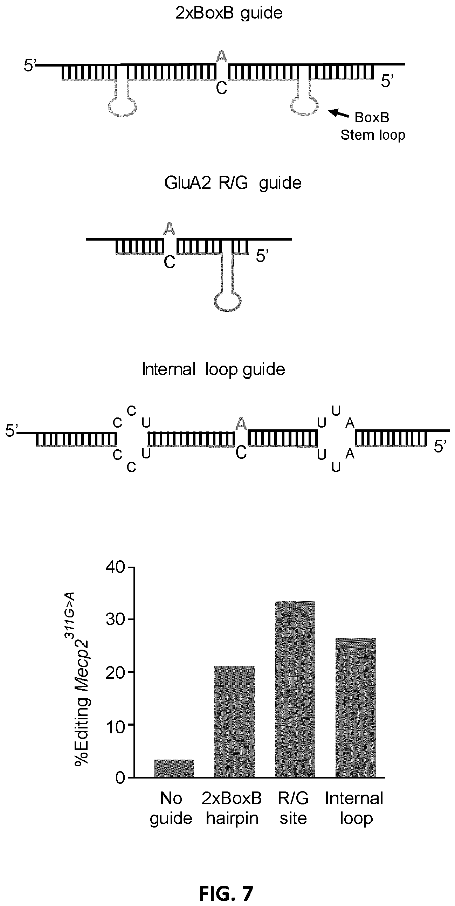

Figure 7 provides schematics of various guide RNA and a graph of the percent editing of *Mecp2^{317G>A}* (*Mecp2^{R106Q}*) in HEK cells without treatment or treated with a guide RNA with 2 BoxB stem loops, a guide RNA comprising a R/G binding site from GluA2, or a guide RNA having internal loops. The HEK cells were also transfected with full-length native ADAR2 cDNA under the control of the CMV promoter.

### DETAILED DESCRIPTION OF THE INVENTION

The present invention is based, in part, on the surprising discovery that one can utilize site-directed RNA editing to repair, at the RNA level (e.g., mRNA), a disease-causing point mutation, e.g., a guanosine to adenosine (G > A) mutation in the Methyl CpG Binding Protein 2 (MECP2) DNA binding domain gene which underlies Rett syndrome. Importantly, this site-directed RNA editing is useful, *inter alia,* to repair an endogenous RNA and restore protein function. Accordingly, in embodiments, the present invention relates to compositions and methods for site-directed RNA editing.

Mice engineered with mutations in *Mecp2* that cause Rett syndrome in humans, either germ line or confined to neural cells, exhibit growth abnormalities, anxiety, and motor deficits, which are similar to Rett syndrome patients (Guy, et al. (2001) Nat. Genet., 27:322-326; Lioy, et al. (2011) Nature 475:497-500; Chen, et al. (2001) Nat. Genet., 27:327-331). Studies in mice indicate that the most robust Rett syndrome phenotypes are neurological, affecting both neurons and glia (Lioy, et al. (2011) Nature 475:497-500; Luikenhuis, et al. (2004) Proc. Natl. Acad. Sci., 101:6033-6038), although many other tissues are also likely affected (Ross, et al. (2016) Hum. Mol. Genet., 25:4389-4404). As in humans, male Rett mice have a more severe disease than female mice. For example, female Rett mice live a normal lifespan, while male mice die between 3 and 4 months of age (Guy, et al. (2001) Nat. Genet., 27:322-326; Chen, et al. (2001) Nat. Genet., 27:327-331). At the cellular level, neural cells in Rett male and female mice have smaller somas, nuclei, and reduced process complexities (Belichenko, et al. (2009) Neurobiol. Dis., 34:71-77; Belichenko, et al. (2009) J. Comp. Neurol., 514:240-258; Fukuda, et al. (2005) J. Neuropathol. Exp. Neurol., 64:537-544; Kishi, et al. (2004) Mol. Cell. Neurosci., 27:306-321; Robinson, et al. (2012) Brain 135:2699-2710; Tropea, et al. (2009) Proc. Natl. Acad. Sci., 106:2029-2034; Stuss, et al. (2012) PLoS One 7:e31896), reminiscent of affected human cells (Armstrong, et al. (1995) J. Neuropathol. Exp. Neurol., 54:195-201; Li, et al. (2013) Cell Stem Cell 13:446-458; Belichenko, et al. (1994) Neuroreport., 5:1509-1513; Bauman, et al. (1995) Neurology 45:1581-1586). Importantly, restoration of MeCP2 inMecp2-null mice, via conditional Cre recombinase (Guy, et al. (2007) Science 315:1143-1147) or gene therapy approaches (Sinnett, et al. (2017) Mol. Ther. Methods Clin. Dev., 5:106-115; Gadalla, et al. (2017) Mol. Ther. Methods Clin. Dev., 5:180-190; Garg, et al. (2013) J. Neurosci., 33:13612-13620; Gadalla, et al. (2013) Mol. Ther., 21:18-30), reverses many of the Rett-like symptoms and cellular deficits, even in late stages of the disease. The phenotype reversals indicate that in humans, Rett syndrome can be treated with gene replacement strategies (Robinson, et al. (2012) Brain 135:2699-2710; Sinnett, et al. (2017) Mol. Ther. Methods Clin. Dev., 5:106-115; Gadalla, et al. (2017) Mol. Ther. Methods Clin. Dev., 5:180-190; Garg, et al. (2013) J. Neurosci., 33:13612-13620; Gadalla, et al. (2013) Mol. Ther., 21:18-30). However, duplications spanning the *MECP2* gene in humans result in MeCP2 overexpression and a severe neurological disorder (Van Esch, et al. (2005) Am. J. Hum. Genet., 77:442-453). Further, MeCP2 in mice is expressed to different levels in different neural cell types and loss of MeCP2 function in mice results in cell-specific alterations in gene expression (Skene, et al. (2010) Mol. Cell., 37:457-468; Ballas, et al. (2009) Nat. Neurosci., 12:311-317; Shahbazian, et al. (2002) Hum. Mol. Genet., 11:115-124; Sugino, et al. (2014) J. Neurosci., 34:12877-12883; Linhoff, et al. (2015) Cell 163:246-255). These findings underscore the challenges for MECP2 gene replacement that must be finely tuned to restore normal MeCP2 levels and cellular physiology across diverse cell types in the nervous system.

Herein, it is shown that repairing *MECP2* mutations at the level of RNA circumvents the problems of both MECP2 overexpression and cell type-specific regulation as the RNA is repaired in the context of the normal transcript. Guanosine to adenosine (G > A) mutations that underlie Rett syndrome (Fyfe, et al. (2003) J. Child. Neurol., 18:709-713) were targeted. A family of naturally occurring enzymes, Adenosine Deaminase Acting on RNA (ADAR), hydrolytically deaminates A to inosine (I) (Bass, et al. (1988) Cell 55:1089-1098; Bass, et al. (1987) Cell 48:607-613; Melcher, et al. (1996) Nature 379:460-464; O'Connell, et al. (1998) Methods 15:51-62; Kim, et al. (1994) Proc. Natl. Acad. Sci., 91:11457-11461) in endogenous mRNAs. Inosine base pairs with cytosine (C) and is translated by the ribosome as G (Basilio, et al. (1962) Proc. Natl. Acad. Sci., 48:613-616). One ADAR family member, ADAR2, is expressed to high levels in brain where it post-transcriptionally alters protein functions, such as ion channel permeability, through deamination of the primary transcript (Bhalla, et al. (2004) Nat. Struct. Mol. Biol., 11:950-956; Sommer, et al. (1991) Cell 67:11-19; Burns, et al. (1997) Nature 387:303-308). In addition to its catalytic activity, natural editing by ADAR2 requires recognition of a double-stranded RNA structure, mediated by an intron in the pre-mRNA, which appropriately positions the target A in an exon for editing (Bhalla, et al. (2004) Nat. Struct. Mol. Biol., 11:950-956; Dawson, et al. (2004) J. Biol. Chem., 279:4941-4951; Higuchi, et al. (1993) Cell 75:1361-1370; Maas, et al. (1996) J. Biol. Chem., 271:12221-12226; Lomeli, et al. (1994) Science 266:1709-1713; Yang, et al. (1997) Proc. Natl. Acad. Sci., 94:4354-4359). A cloned catalytic domain in human ADAR2 (hADAR2) has been harnessed, in various configurations, to target G > A repair in heterologously expressed mRNAs, usually at stop codons (Hanswillemenke, et al. (2015) J. Am. Chem. Soc., 137:15875-15881; Vogel, et al. (2014) ChemMedChem 9:2021-2025; Vogel, et al. (2014) Angew Chem. Int. Ed. Engl., 53:6267-6271; Schneider, et al. (2014) Nucleic Acids Res., 42:e87; Montiel-González, et al. (2016) Nucleic Acids Res., 44:e157; Montiel-Gonzalez, et al. (2013) Proc. Natl. Acad. Sci., 110:18285-18290). In one approach, the native RNA binding domains in ADAR2 are replaced with an RNA binding peptide from bacteriophage lambda (AN; Montiel-Gonzalez, et al. (2013) Proc. Natl. Acad. Sci., 110:18285-18290) that binds to a specific short RNA hairpin with nanomolar affinity (Austin, et al. (2002) J. Am. Chem. Soc., 124:10966-10967). Targeted editing of heterologous mRNA is then achieved by expression of the hybrid ADAR2 protein along with an RNA guide that contains the λN-recognized stem loops and a region complementary to the target mRNA (Montiel-González, et al. (2016) Nucleic Acids Res., 44:e157; Montiel-Gonzalez, et al. (2013) Proc. Natl. Acad. Sci., 110:18285-18290).

Previously, no endogenous RNAs or mRNAs have been repaired by site-directed RNA editing, particularly with regard to being repaired to yield a functional protein However, this approach for G > A mutations in endogenous *Mecp2* is demonstrated herein for the first time. The mutations in *Mecp2* are in domains that encode well-established functions. Additionally, the fidelity of repair can be monitored by sequence analysis, Western blotting, and, at the single cell level, immunochemistry. Here, the recombinant hADAR2-λN protein (also referred to herein as Editase) was used for effective repair of G > A mutations within endogenous *Mecp2* transcripts. After determining parameters for *Mecp2* editing in transfected mouse neuroblastoma (N2A) cells, an adeno-associated virus (AAV) was used to transduce primary neuronal cultures from a Rett syndrome mouse model that contains a severe human G > A mutation in the DNA binding domain (MeCP2^{317G>A}; MeCP2^{R106Q}). This mutation results in reduced MeCP2 protein levels and greatly attenuated binding to heterochromatin. Editing efficiency of the mutant RNA was first quantitated in neurons and then it was tested whether editing rescues MeCP2 protein levels and leads to enrichment of binding in heterochromatin foci, a key property of MeCP2 in cells, including neurons, glia, and non-neuronal cell types. The results presented herein show that site-directed RNA editing can therapeutically repair disease-causing MECP2 mutations underlying Rett syndrome as well as other neurological diseases amenable to gene therapy.

In accordance with the instant invention, methods of editing a nucleic acid molecule in a cell are provided. In a particular embodiment, the nucleic acid molecule to be edited is an RNA molecule, particularly an RNA molecule within the nucleus (e.g., a primary transcript, pre-mRNA, or mRNA (e.g., an mRNA prior to transport out of the nucleus)). In a particular embodiment, the nucleic acid molecule to be edited is endogenous and/or a nuclear transcript. With gene editing, such as with CRISPR technology, off-target mutations in the genome are permanent. In contrast, RNA turnover in the cells means that off-target mutations within the RNA will be temporary. Additionally, RNA editing, unlike CRISPR genomic editing, may be graded. Consequently, off-target mutations are not necessarily edited to 100%.

In embodiments, the present invention provides for methods of RNA editing a nucleic acid molecule that provides fine-tuning of protein expression and/or function. For instance, the methods of the present invention allow for restoration of normal levels of protein expression and/or function relative to an unedited state. In the context of the treatments described herein, the methods of the present invention allow for restoration of normal levels or at least near normal levels of protein expression and/or function relative to an untreated state.

In embodiments, the present invention provides for methods of RNA editing a nucleic acid molecule that provides, e.g. in the context of the described therapies, transient editing that is tunable (e.g. via dosing). For instance, the present invention allows for a reversible editing of a target RNA.

In a particular embodiment, the cells being edited are non-dividing cells. In a particular embodiment, the cells being edited are neurons and/or glial cells. In a particular embodiment, the cells being edited are non-neuronal cells. In a particular embodiment, the cells being edited are neurons. The cells (e.g., neurons) may be in the central nervous system (e.g., brain, spinal cord) and/or peripheral nervous system. The cells may be in a subject to be treated (e.g., an *in vivo* method of treatment) or the cells may be treated *in vitro* and then administered to a subject (e.g., an *ex vivo* method of treatment).

In certain embodiments of the instant invention, the methods comprise delivering 1) a nucleic acid molecule encoding an RNA editing enzyme linked or fused to an RNA binding domain and 2) a guide RNA or a nucleic acid molecule encoding the guide RNA to a cell. In a particular embodiment, the RNA binding domain is linked to the N-terminus of the RNA editing enzyme. In embodiments, the fusion comprising the RNA editing enzyme and the RNA binding domain does not comprise at least one nuclear localization signal (NLS). In embodiments, the fusion comprising the RNA editing enzyme and the RNA binding domain further comprises at least one nuclear localization signal (NLS). For example, the fusion comprising the RNA editing enzyme and the RNA binding domain further comprises one, two, three, four, five, or more NLSs. When multiple NLSs are employed, each NLS may be linked directly to each other or separated by an amino acid linker of 1 to about 5 amino acids. In a particular embodiment, the NLSs are at the N-terminus of the fusion protein. Examples of NLS are provided in Kosugi et al. (J. Biol. Chem. (2009) 284:478-485; incorporated by reference herein). In a particular embodiment, the NLS comprises the consensus sequence K(K/R)X(K/R) (e.g., a monopartite NLS). In a particular embodiment, the NLS comprises the consensus sequence (K/R)(K/R)X₁₀₋₁₂(K/R)_{3/5}, where (K/R)_{3/5} represents at least three of the five amino acids is either lysine or arginine. In a particular embodiment, the NLS comprises the c-myc NLS. In a particular embodiment, the c-myc NLS comprises the sequence PAAKRVKLD (SEQ ID NO: 54). In a particular embodiment, the NLS is the nucleoplasmin NLS. In a particular embodiment, the nucleoplasmin NLS comprises the sequence KRPAATKKAGQAKKKK (SEQ ID NO: 60). In a particular embodiment, the NLS comprises the SV40 Large T-antigen NLS. In a particular embodiment, the SV40 Large T-antigen NLS comprises the sequence PKKKRKV (SEQ ID NO: 47). In a particular embodiment, the fusion comprises three SV40 Large T-antigen NLSs (e.g., the sequence DPKKKRKVDPKKKRKVDPKKKRKV (SEQ ID NO: 67)). In various embodiment, the NLS may comprise mutations/variations in the above sequences (e.g., K(K/R)X(K/R), (K/R)(K/R)X₁₀₋₁₂(K/R)_{3/5}, SEQ ID NOs: 60, 47, 54, or 67) such that they contain 1 or more substitutions, additions or deletions (e.g. about 1, or about 2, or about 3, or about 4, or about 5, or about 10, or about 15, including about 1 to 5, or about 1 to 10, or about 1 to 15 substitutions, additions, or deletions). In a particular embodiment, the lysine amino acids within the NLS may be substituted with arginine amino acids and/or the arginine amino acids within the NLS may be substituted with lysine amino acids. The fusion protein may further comprise a purification tag (e.g., an HA tag), optionally at the N-terminus of the fusion protein.

The nucleic acid molecules of the instant invention may be contained within a single vector or contained in separate vectors. For example, the nucleic acid molecule encoding an RNA editing enzyme linked or fused to an RNA binding domain and the nucleic acid molecule encoding the guide RNA are contained within a single vector. The nucleic acid molecules may be delivered to the cell consecutively (before or after) and/or at the same time (concurrently). The nucleic acid molecules may be delivered in the same composition or in separate compositions (e.g., when contained in separate vectors). In a particular embodiment, the nucleic acid molecules are delivered in a single vector, particularly a viral vector such as an AAV vector.

In a particular embodiment, the RNA editing enzyme is human. In a particular embodiment, the RNA editing enzyme is a deaminase. Examples of deaminases include, without limitation, Adenosine Deaminase Acting on RNA (ADAR), apolipoprotein B mRNA editing enzyme, catalytic polypeptide-like (APOBEC (e.g., APOBEC1, APOBEC3A, APOBEC3G)), and activation-induced cytidine deaminase (AICDA or AID; C:G is converted to a T:A). In a particular embodiment, the RNA editing enzyme is an ADAR, such as ADAR1, ADAR2, or ADAR3. In a particular embodiment, the RNA editing enzyme is ADAR1 (see, e.g., Gene ID: 103 and GenBank Accession Nos. NM_001111.5 and NP_001102.3 and isoforms thereof). In a particular embodiment, the RNA editing enzyme is ADAR2. The RNA editing enzyme may be less than full length. In a particular embodiment, the RNA editing enzyme lacks its natural RNA binding domain. For example, the RNA editing enzyme may comprise or consists of the catalytic domain of the enzyme.

An example of the amino acid sequence of human ADAR1 is: 
MNPRQGYSLS GYYTHPFQGY EHRQLRYQQP GPGSSPSSFL LKQIEFLKGQ LPEAPVIGKQ
TPSLPPSLPG LRPRFPVLLA SSTRGRQVDI RGVPRGVHLR SQGLQRGFQH PSPRGRSLPQ
RGVDCLSSHF QELSIYQDQE QRILKFLEEL GEGKATTAHD LSGKLGTPKK EINRVLYSLA
KKGKLQKEAG TPPLWKIAVS TQAWNQHSGV VRPDGHSQGA PNSDPSLEPE DRNSTSVSED
LLEPFIAVSA QAWNQHSGVV RPDSHSQGSP NSDPGLEPED SNSTSALEDP LEFLDMAEIK
EKICDYLFNV SDSSALNLAK NIGLTKARDI NAVLIDMERQ GDVYRQGTTP PIWHLTDKKR
ERMQIKRNTN SVPETAPAAI PETKRNAEFL TCNIPTSNAS NNMVTTEKVE NGQEPVIKLE
NRQEARPEPA RLKPPVHYNG PSKAGYVDFE NGQWATDDIP DDLNSIRAAP GEFRAIMEMP
SFYSHGLPRC SPYKKLTECQ LKNPISGLLE YAQFASQTCE FNMIEQSGPP HEPRFKFQVV
INGREFPPAE AGSKKVAKQD AAMKAMTILL EEAKAKDSGK SEESSHYSTE KESEKTAESQ
TPTPSATSFF SGKSPVTTLL ECMHKLGNSC EFRLLSKEGP AHEPKFQYCV AVGAQTFPSV
SAPSKKVAKQ MAAEEAMKAL HGEATNSMAS DNQPEGMISE SLDNLESMMP NKVRKIGELV
RYLNTNPVGG LLEYARSHGF AAEFKLVDQS GPPHEPKFVY QAKVGGRWFP AVCAHSKKQG
KQEAADAALR VLIGENEKAE RMGFTEVTPV TGASLRRTML LLSRSPEAQP KTLPLTGSTF
HDQIAMLSHR CFNTLTNSFQ PSLLGRKILA AIIMKKDSED MGVVVSLGTG NRCVKGDSLS
LKGETVNDCH AEIISRRGFI RFLYSELMKY NSQTAKDSIF EPAKGGEKLQ IKKTVSFHLY
ISTAPCGDGA LFDKSCSDRA MESTESRHYP VFENPKQGKL RTKVENGEGT IPVESSDIVP
TWDGIRLGER LRTMSCSDKI LRWNVLGLQG ALLTHFLQPI YLKSVTLGYL FSQGHLTRAI
CCRVTRDGSA FEDGLRHPFI VNHPKVGRVS IYDSKRQSGK TKETSVNWCL ADGYDLEILD
GTRGTVDGPR NELSRVSKKN IFLLFKKLCS FRYRRDLLRL SYGEAKKAAR DYETAKNYFK
KGLKDMGYGN WISKPQEEKN FYLCPV (SEQ ID NO: 72)
 In a particular embodiment, the deaminase domain of ADAR1 comprises amino acids 839-1222 of SEQ ID NO: 72. In a particular embodiment, the RNA editing enzyme comprises a sequence which has at least 80%, 85%, 90%, 95%, 97%, 99%, or 100% homology or identity, particularly at least 95%, 97%, 99%, or 100% homology or identity, to SEQ ID NO: 72 of the deaminase domain thereof.

In a particular embodiment, the RNA editing enzyme comprises the deaminase domain of human ADAR2. In a particular embodiment, the deaminase domain of human ADAR2 comprises amino acids 299-701 of GenBank Accession No. U82120. In a particular embodiment, the ADAR2 or deaminase domain thereof comprises the E488Q mutation (Montiel-González, et al. (2016) Nucleic Acids Res., 44:e157). In a particular embodiment, the deaminase domain of human ADAR2 comprises

LHLDQTPSRQPIPSEGLQLHLPQVLADAVSRLVLGKFGDLTDNFSSPHAR
RKVLAGVVMTTGTDVKDAKVISVSTGTKCINGEYMSDRGLALNDCHAEII
SRRSLLRFLYTQLELYLNNKDDQKRSIFQKSERGGFRLKENVQFHLYIST
SPCGDARIFSPHEPILEEPADRHPNRKARGQLRTKIESGEGTIPVRSNAS
IQTWDGVLQGERLLTMSCSDKIARWNVVGIQGSLLSIFVEPIYFSSIILG
SLYHGDHLSRAMYQRISNIEDLPPLYTLNKPLLSGISNAEARQPGKAPNF
SVNWTVGDSAIEVINATTGKDELGRASRLCKHALYCRWMRVHGKVPSHLL
RSKITKPNVYHESKLAAKEYQAAKARLFTAFIKAGLGAWVEKPTEQDQFS
LTP (SEQ ID NO: 55; E488 is indicated).

In a particular embodiment, the deaminase domain of human ADAR2 comprises 
LHLDQTPSRQPIPSEGLQLHLPQVLADAVSRLVLGKFGDLTDNFSSPHAR
RKVLAGVVMTTGTDVKDAKVISVSTGTKCINGEYMSDRGLALNDCHAEII
SRRSLLRFLYTQLELYLNNKDDQKRSIFQKSERGGFRLKENVQFHLYIST
SPCGDARIFSPHEPILEEPADRHPNRKARGQLRTKIESGQGTIPVRSNAS
IQTWDGVLQGERLLTMSCSDKIARWNVVGIQGSLLSIFVEPIYFSSIILG
SLYHGDHLSRAMYQRISNIEDLPPLYTLNKPLLSGISNAEARQPGKAPNF
SVNWTVGDSAIEVINATTGKDELGRASRLCKHALYCRWMRVHGKVPSHLL
 
RSKITKPNVYHESKLAAKEYQAAKARLFTAFIKAGLGAWVEKPTEQDQFS
LTP    (SEQ ID NO: 71; E488Q is indicated).
 In a particular embodiment, the RNA editing enzyme comprises a sequence which has at least 80%, 85%, 90%, 95%, 97%, 99%, or 100% homology or identity, particularly at least 95%, 97%, 99%, or 100% homology or identity, to SEQ ID NO: 55 or 71.

As stated hereinabove, the RNA editing enzyme is linked to an RNA binding domain. The RNA editing enzyme may be linked directly (i.e., no linker sequence) to the RNA binding domain or may be linked via a polypeptide linker. For example, the polypeptide linker may comprise 1 to about 50 amino acids, 1 to about 25 amino acids, 1 to about 20 amino acids, 1 to about 15 amino acids, 1 to about 10 amino acids, or 1 to about 5 amino acids. In a particular embodiment, the linker comprises the sequence (GGGGS)ₙ (SEQ ID NO: 44), wherein n is 1 to about 10, particularly 1 to about 5. For example, the linker sequence may be: GGGGSGGGGSGGGGS (SEQ ID NO: 45). In various embodiment, the linker may comprise mutations/variations in the above sequences (e.g., SEQ ID NOs: 44 or 45) such that they contain 1 or more substitutions, additions or deletions (e.g. about 1, or about 2, or about 3, or about 4, or about 5, or about 10, or about 15, including about 1 to 5, or about 1 to 10, or about 1 to 15 substitutions, additions, or deletions).

The RNA binding domain can be any polypeptide that specifically recognizes a particular RNA sequence and/or RNA structure (e.g., hairpin). In a particular embodiment, the RNA binding domain is an artificial RNA binding domain, particularly one with a high affinity for RNA. In a particular embodiment, the RNA binding domain is a phage RNA binding domain (see, e.g., Keryer-Bibens, et al., Biol. Cell (2008) 100:125-138). For example, the RNA binding domain is the λN peptide (see, e.g., Cilley, et al., RNA (1997) 3:57-67) or phage MS2 coat protein (see, e.g., Johansson, et al. Sem. Virol. (1997) 8:176-185). In a particular embodiment, the λN peptide comprises the amino acid sequence: MNARTRRRERRAEKQAQWKAAN (SEQ ID NO: 46). In a particular embodiment, the λN peptide comprises a sequence which has at least 80%, 85%, 90%, 95%, 97%, 99%, or 100% homology or identity, particularly at least 95%, 97%, 99%, or 100% homology or identity, to SEQ ID NO: 46.

In embodiments, the fusion protein comprises the λN peptide (SEQ ID NO: 46) linked via an amino acid linker to the amino terminus of the deaminase domain of human ADAR2 (e.g., SEQ ID NO: 55 or 71). In a particular embodiment, the linker comprises the sequence (GGGGS)ₙ (SEQ ID NO: 44; wherein n is 1 to about 10 or 1 to about 5) or the sequence GGGGSGGGGSGGGGS (SEQ ID NO: 45). In a particular embodiment, the fusion protein comprises the sequence: 
MNARTRRRERRAEKQAQWKAANGGGGSGGGGSGGGGSLHLDQTPSRQPIP
SEGLQLHLPQVLADAVSRLVLGKFGDLTDNFSSPHARRKVLAGVVMTTGT
DVKDAKVISVSTGTKCINGEYMSDRGLALNDCHAEIISRRSLLRFLYTQL
ELYLNNKDDQKRSIFQKSERGGFRLKENVQFHLYISTSPCGDARIFSPHE
PILEEPADRHPNRKARGQLRTKIESGEGTIPVRSNASIQTWDGVLQGERL
LTMSCSDKIARWNVVGIQGSLLSIFVEPIYFSSIILGSLYHGDHLSRAMY
QRISNIEDLPPLYTLNKPLLSGISNAEARQPGKAPNFSVNWTVGDSAIEV
INATTGKDELGRASRLCKHALYCRWMRVHGKVPSHLLRSKITKPNVYHES
KLAAKEYQAAKARLFTAFIKAGLGAWVEKPTEQDQFSLTP (SEQ ID NO: 68).
 In a particular embodiment, the fusion protein further comprises at least one NLS at the N-terminus. In a particular embodiment, NLS comprises the SV40 Large T-antigen NLS (e.g., SEQ ID NO: 47). In a particular embodiment, the fusion comprises three SV40 Large T-antigen NLSs (e.g., SEQ ID NO: 67). In a particular embodiment, the fusion protein comprises the sequence: 
DPKKKRKVDPKKKRKVDPKKKRKVMNARTRRRERRAEKQAQWKAANGGGG
SGGGGSGGGGSLHLDQTPSRQPIPSEGLQLHLPQVLADAVSRLVLGKFGD
LTDNFSSPHARRKVLAGVVMTTGTDVKDAKVISVSTGTKCINGEYMSDRG
LALNDCHAEIISRRSLLRFLYTQLELYLNNKDDQKRSIFQKSERGGFRLK
ENVQFHLYISTSPCGDARIFSPHEPILEEPADRHPNRKARGQLRTKIESG
EGTIPVRSNASIQTWDGVLQGERLLTMSCSDKIARWNVVGIQGSLLSIFV
EPIYFSSIILGSLYHGDHLSRAMYQRISNIEDLPPLYTLNKPLLSGISNA
EARQPGKAPNFSVNWTVGDSAIEVINATTGKDELGRASRLCKHALYCRWM
RVHGKVPSHLLRSKITKPNVYHESKLAAKEYQAAKARLFTAFIKAGLGAW
VEKPTEQDQFSLTP (SEQ ID NO: 69).
 In a particular embodiment, the fusion protein comprises a sequence which has at least 80%, 85%, 90%, 95%, 97%, 99%, or 100% homology or identity, particularly at least 95%, 97%, 99%, or 100% homology or identity, to SEQ ID NO: 68 or 69.

The guide RNA of the instant invention comprises a sequence which targets or specifically hybridizes with a target sequence (e.g., complementary sequence) and a sequence recognized by the RNA binding domain. The guide RNA comprises a mismatch with the target sequence directed to the nucleotide (e.g., adenosine) to be changed or edited. As used herein, the term "specifically hybridizes" does not mean that the nucleic acid molecule needs to be 100% complementary to the target sequence. Rather, the sequence - not including the mismatch nucleotide(s) to be changed/edited - may be at least 80%, 85%, 90%, 95%, 97%, 99%, or 100% complementary, particularly at least 95%, 97%, 99%, or 100% complementary, to the target sequence. However, as explained herein, the sequence may comprise additional mismatches (e.g., a G mismatch) to reduce off-targeting editing. In embodiments, the sequence may contain about 1, or about 2, or about 3, or about 4, or about 5, or about 10, or about 15, including about 1 to 5, or about 1 to 10, or about 1 to 15 mismatches. In a particular embodiment, the region of complementarity (e.g., between a guide RNA and a target sequence) is at least about 10, at least about 12, at least about 15, at least about 17, at least about 20, at least about 25, at least about 30, at least about 35, or more nucleotides. In a particular embodiment, the region of complementarity (e.g., between a guide RNA and a target sequence) is about 15 to about 30 nucleotides, about 15 to about 25 nucleotides, about 20 to about 30 nucleotides, about 20 to about 25 nucleotides, or about 20, 21, 22, 23, 24, or 25 nucleotides. Typically, the mismatch between the guide RNA and the target sequence will be towards the middle (e.g., within the middle 50% of the guide RNA sequence) of the region of complementarity between the guide RNA and the target sequence. In a particular embodiment, the target sequence comprises SEQ ID NO: 52.

The guide RNA comprises the correct or desired edit to the RNA in the cell. The guide RNA can be used to correct any mutation, particularly a point mutation, including missense mutations and nonsense mutations. For example, with Rett syndrome, there are common G>A mutations. These mutations result in amino acid changes R106Q (CAA), W104X (UAG), and R306H (CAC). In the case of nonsense mutations, these may be a C to T mutation with an A in the 3' position to form the stop codon or in the middle position (e.g., UAG to UGG). The nonsense mutations can be edited to remove the stop codon. In a particular embodiment, the guide RNA comprises a C to match the A of these mutants so that the A can be deaminated to I (e.g., by an ADAR).

The guide RNA of the instant invention comprises one or more sequences recognized by the RNA binding domain. In a particular embodiment, the guide RNA comprises two sequences recognized by the RNA binding domain. In a particular embodiment, the two sequences recognized by the RNA binding domain can be on both sides of the mismatch or the sequence which specifically hybridizes with the target sequence. For example, one sequence recognized by RNA binding domain (e.g., BoxB) is located about 15-20 or about 16-18 nucleotides 5' of the target mutation and a second sequence recognized by RNA binding domain (e.g., BoxB) is located about 8-12 or about 10 nucleotides 3' of the target mutation. In a particular embodiment, the sequences recognized by the RNA binding domain are not at the termini of the guide RNA (i.e., sequences at the termini of the guide RNA may be complementary to the target sequence). When two or more sequences recognized by the RNA binding domain are present, the sequences can be the same or different - although they are preferably recognized by the same RNA binding domain. In a particular embodiment, the sequence recognized by the RNA binding domain is a BoxB sequence. In a particular embodiment, the BoxB sequence comprises GCCCUGAAAAAGGGC (SEQ ID NO: 48) or GGCCCUGAAAAAGGGCC (SEQ ID NO: 49). In a particular embodiment, the BoxB sequence has at least 80%, 85%, 90%, 95%, 97%, 99%, or 100% homology or identity, particularly at least 95%, 97%, 99%, or 100% homology or identity, to SEQ ID NO: 48 or 49.

In a particular embodiment, the guide RNA targets a sequence or comprises a sequence (inclusive of RNA version of DNA molecules) as set forth in Table 1. In a particular embodiment, the guide RNA targets a sequence or comprises a sequence which has at least 80%, 85%, 90%, 95%, 97%, 99%, or 100% homology or identity, particularly at least 95%, 97%, 99%, or 100% homology or identity, to a sequence set forth in Table 1. In a particular embodiment, the guide RNA comprises one of SEQ ID NOs: 15-22, particularly SEQ ID NO: 15, 17, 19, or 21, or comprises a sequence which has at least 80%, 85%, 90%, 95%, 97%, 99%, or 100% homology or identity, particularly at least 95%, 97%, 99%, or 100% homology or identity, one of SEQ ID NOs: 15-22, particularly SEQ ID NO: 15, 17, 19, or 21.

Nucleic acid molecules comprising the nucleic acid sequence encoding the guide RNA may comprise multiple copies of the nucleic acid sequence encoding the guide RNA. For example, the nucleic acid molecule may comprise 1, 2, 3, 4, 5, 6, 7, 8, 9, 10, or more copies of the nucleic acid sequence encoding the guide RNA, each under the control of a promoter.

In a particular embodiment, the nucleic acid molecules of the instant invention are delivered (e.g., via infection, transfection, electroporation, etc.) and expressed in cells via a vector (e.g., a plasmid), particularly a viral vector. The expression vectors of the instant invention may employ a strong promoter, a constitutive promoter, tissue or cell specific promoter, ubiquitous promoter, and/or a regulated promoter. In a particular embodiment, the promoter for the nucleic acid molecule encoding an RNA editing enzyme linked or fused to an RNA binding domain is a tissue or cell specific promoter. In a particular embodiment, the promoter is a neuron specific promoter or a ubiquitous promoter. Examples of promoters are well known in the art and include, but are not limited to, a synapsin promoter, particularly the *Synapsin* 1 promoter, the CAG promoter, and the *MECP2* promoter. With regard to the guide RNA, examples of RNA promoters are well known in the art and include, but are not limited to, RNA polymerase III promoters (e.g., U6 and H1; see, e.g., Myslinski et al. (2001) Nucl. Acids Res., 29:2502-09) or other promoters known to express short RNAs. In a particular embodiment, the promoter is the human U6 promoter. Examples of expression vectors for expressing the molecules of the invention include, without limitation, plasmids and viral vectors (e.g., adeno-associated viruses (AAVs), adenoviruses, retroviruses, and lentiviruses). In a particular embodiment, the vector is an AAV (e.g., AAV-1 to AAV-12 and other serotypes and hybrid AAV vectors; e.g. AAV1, or AAV2, or AAV3, or AAV4, or AAV5, or AAV6, or AAV7, or AAV8, or AAV9, or AAV10, or AAV11, or AAV12). In a particular embodiment, the vector is capable of infecting neurons and/or glia.

In accordance with another aspect, the methods of the instant invention, including the RNA editing and therapeutic methods, comprise delivering a guide RNA or a nucleic acid molecule encoding the guide RNA to a cell, as described hereinabove, but without a nucleic acid molecule encoding an RNA editing enzyme linked or fused to an RNA binding domain. ADARs (e.g., ADARs 1-3) are expressed to high levels in the nervous system. Thus, the guide RNA of the instant invention can attract the endogenous deaminase enzymes such as ADAR enzymes, particularly ADAR1 or ADAR2, to endogenous *MECP2* RNA. Thus, in embodiments, the present invention allows for engagement of endogenous ADAR enzymes and does not require recombinant ADAR enzymes. By using only the guide RNA, any potential immune response to non-mammalian RNA binding domains is avoided. Further, the method allows for highly iterative guide sequences to be contained on the AAV vectors and diminishes off target editing. In a particular embodiment of this aspect of the instant invention, the guide comprises a sequence which targets or specifically hybridizes with a target sequence (e.g., complementary sequence) and a sequence recognized by a deaminase, particularly an ADAR (e.g., ADAR1 or ADAR2). The guide RNA may comprise, without limitation, an RNA hairpin (e.g., based on natural targets of ADARs), mismatches that create double stranded "bulges" recognized by ADARs, and/or any other sequences that are required normally for on target editing by endogenous ADARs. In a particular embodiment, the guide RNA comprises one, two, or more BoxB sequences. In a particular embodiment, the guide RNA comprises a R/G binding site from GluR2 (Wettengel, et al. (2017) Nucleic Acids Res., 45(5): 2797-2808; Fukuda, et al. (2017) Sci. Rep.,7:41478; e.g., comprising GUGGAAUAGUAUAACAA-UAUGCUAAAUGUUGUUAUAGUAUCCCAC (SEQ ID NO: 70) or a sequence which has at least 80%, 85%, 90%, 95%, 97%, 99%, or 100% homology or identity, particularly at least 95%, 97%, 99%, or 100% homology or identity). In a particular embodiment, the guide RNA comprises having internal loops (Lehmann, et al. (1999) J. Mol. Biol., 291(1):1-13; e.g., loops comprising 4, 6, 8, 10, or more nucleotides). In a particular embodiment, guide RNA comprises a region complementary to *MECP2* RNA, the mismatch (e.g., A:C) for editing, and, optionally, A:G mismatches for off target editing. In a particular embodiment, the nucleic acid molecule encoding the guide RNA is contained within a vector as described herein. In a particular embodiment, the guide RNA is expressed from the U6 promoter or other promoters that express small RNAs.

In accordance with the instant invention, methods of treating, inhibiting, and/or preventing a genetic disease of the central nervous system are provided. In accordance with the instant invention, methods of treating, inhibiting, and/or preventing a progressive neurodevelopmental disease are provided. For instance, the present invention provides, in embodiments, treatment, inhibition, and/or prevention of a genetic central nervous system disease characterized by a mutation in a subject's RNA. In embodiments, the present invention provides for methods of treating, inhibiting, and/or preventing a progressive neurodevelopmental disease by restoring, directly or indirectly, the translation of an RNA to a normal protein due to the restoration of an aberrant G mutation, e.g. by editing the RNA to be read as having a G.

In accordance with the instant invention, methods of treating, inhibiting, and/or preventing a genetic disease of the central nervous system, including a progressive neurodevelopmental disease, including Rett syndrome are effective *in vivo* or *ex vivo.* For instance, for *in vivo* methods, the present nucleic acid molecule(s) (e.g. encoding a described fusion protein, e.g. encoding a described guide RNA) is/are administered to a subject. For *ex vivo* methods, cells (syngeneic or allogenic) are contacted with the present nucleic acid molecule (e.g. encoding a described fusion protein, e.g. encoding a described guide RNA) and introduced into a subject. Embodiments relating to treatment apply equally to *in vivo* methods and *ex vivo* methods.

In accordance with the instant invention, methods of treating, inhibiting, and/or preventing a genetic disease associated with the MECP2 gene are provided. In embodiments, the present invention provides genetic editing, e.g. at the RNA level, to restore levels of functional MECP2 protein to that of an undiseased or healthy subject. The Mecp2 may be human or mouse, particularly human. In embodiments, the present invention provides genetic editing, e.g. at the RNA level, to increase levels of functional MECP2 protein relative to a diseased state. In embodiments, the genetic disease associated with the MECP2 gene is a neonatal encephalopathy, microcephaly, X-linked intellectual disability, PPM-X syndrome (manic depressive (p)sychosis, (p)yramidal signs, (p)arkinsonism, and (m)acro-orchidism), bipolar disorder. parkinsonism, increased muscle tone, exaggerated reflexes, and macroorchidism, or combinations thereof. In embodiments, the genetic disease associated with the MECP2 gene effects a male or female subject.

In accordance with the instant invention, methods of treating, inhibiting, and/or preventing Rett syndrome are provided. In a particular embodiment, the Rett syndrome is characterized by a G>A mutation in MeCP2. For example, the Rett syndrome may be characterized by a R106Q, W104X, or R306H mutation in MECP2. Exemplary amino acid and nucleotide sequences of human MECP2 are provided at GenBank Gene ID 4204 and GenBank Accession Nos. NM _004992.3 and NP_004983.1 (see also isoforms at GenBank Accession Nos. NM_001110792.1, NP_001104262.1, NM 001316337.1, and NP_001303266.1). In a particular embodiment, the Rett syndrome comprises the R106Q mutation in MeCP2. In a particular embodiment, the method comprises administering a nucleic acid molecule encoding an RNA editing enzyme linked or fused to an RNA binding domain and a guide RNA or a nucleic acid molecule encoding the guide RNA. In a particular embodiment, the method comprises administering a guide RNA or a nucleic acid molecule encoding the guide RNA. The nucleic acid molecules may be administered directly to the subject or may be delivered to cells which are then administered to the subject.

In a particular embodiment, the amino acid sequence of MECP2 is: 
MVAGMLGLRE EKSEDQDLQG LKDKPLKFKK VKKDKKEEKE GKHEPVQPSA
HHSAEPAEAG KAETSEGSGS APAVPEASAS PKQRRSIIRD RGPMYDDPTL
PEGWTRKLKQ RKSGRSAGKY DVYLINPQGK AFRSKVELIA YFEKVGDTSL DPNDFDFTVT
GRGSPSRREQ KPPKKPKSPK APGTGRGRGR PKGSGTTRPK
AATSEGVQVK RVLEKSPGKL LVKMPFQTSP GGKAEGGGAT TSTQVMVIKR
PGRKRKAEAD PQAIPKKRGR KPGSVVAAAA AEAKKKAVKE SSIRSVQETV
LPIKKRKTRE TVSIEVKEVV KPLLVSTLGE KSGKGLKTCK SPGRKSKESS PKGRSSSASS
PPKKEHHHHH HHSESPKAPV PLLPPLPPPP PEPESSEDPT SPPEPQDLSS SVCKEEKMPR
GGSLESDGCP KEPAKTQPAV ATAATAAEKY KHRGEGERKD IVSSSMPRPN REEPVDSRTP
VTERVS (SEQ ID NO: 56)
 R106, W104, and R306 are indicated hereinabove with underlining.

In a particular embodiment, the nucleic acid encoding MECP2 is:

atgg tagctgggat gttagggctc agggaagaaa agtcagaaga ccaggacctc cagggcctca
aggacaaacc cctcaagttt aaaaaggtga agaaagataa gaaagaagag aaagagggca
agcatgagcc cgtgcagcca tcagcccacc actctgctga gcccgcagag gcaggcaaag
cagagacatc
agaagggtca ggctccgccc cggctgtgcc ggaagcttct gcctccccca aacagcggcg
ctccatcatc cgtgaccggg gacccatgta tgatgacccc accctgcctg aaggctggac
acggaagctt aagcaaagga aatctggccg ctctgctggg aagtatgatg tgtatttgat
caatccccag ggaaaagcct ttcgctctaa agtggagttg attgcgtact tcgaaaaggt
aggcgacaca tccctggacc ctaatgattt tgacttcacg gtaactggga gagggagccc
ctcccggcga gagcagaaac cacctaagaa gcccaaatct cccaaagctc caggaactgg
cagaggccgg ggacgcccca aagggagcgg caccacgaga cccaaggcgg ccacgtcaga
gggtgtgcag gtgaaaaggg tcctggagaa aagtcctggg aagctccttg tcaagatgcc
ttttcaaact tcgccagggg gcaaggctga gggggtgggg gccaccacat ccacccaggt
catggtgatc aaacgccccg gcaggaagcg aaaagctgag gccgaccctc aggccattcc
caagaaacgg ggccgaaagc cggggagtgt ggtggcagcc gctgccgccg aggccaaaaa
gaaagccgtg aaggagtctt ctatccgatc tgtgcaggag accgtactcc ccatcaagaa
gcgcaagacc cgggagacgg tcagcatcga ggtcaaggaa gtggtgaagc ccctgctggt
gtccaccctc ggtgagaaga gcgggaaagg actgaagacc tgtaagagcc ctgggcggaa
aagcaaggag agcagcccca aggggcgcag cagcagcgcc tcctcacccc ccaagaagga
gcaccaccac catcaccacc actcagagtc cccaaaggcc cccgtgccac tgctcccacc
cctgccccca cctccacctg agcccgagag ctccgaggac cccaccagcc cccctgagcc
ccaggacttg agcagcagcg tctgcaaaga ggagaagatg cccagaggag gctcactgga
gagcgacggc tgccccaagg agccagctaa gactcagccc gcggttgcca ccgccgccac
ggccgcagaa aagtacaaac accgagggga gggagagcgc aaagacattg tttcatcctc
catgccaagg ccaaacagag aggagcctgt ggacagccgg acgcccgtga ccgagagagt
tagctga (SEQ ID NO: 57).

In embodiments, the present invention provides methods of treating, inhibiting, and/or preventing Rett syndrome, including classical Rett syndrome and variant Rett syndrome (a.k.a. atypical Rett syndrome). In embodiments, the Rett syndrome is the Zappella variant, Hanefeld variant, Rolando variant, and/or 'forme fruste' variant.

In embodiments, the present invention provides reduction, amelioration, and/or abrogation of one or more symptoms of Rett syndrome, including, without limitation, ataxia, uncontrolled hand movements (e.g., hand wringing or squeezing, clapping, rubbing, washing, or hand to mouth movements), acquired microcephaly, autistic-like behaviors, breathing irregularities, feeding and swallowing difficulties, growth retardation, hypotonia, panic attacks, teeth grinding (bruxism), tremors, apraxia, heart irregularities (e.g., QT interval and/or T-wave abnormalities), and seizures.

In embodiments, the present compositions may be used in combination with any of the following in the present methods of treating, inhibiting, and/or preventing, e.g. of Rett syndrome: tridecanoic acid, fingolimod (e.g. GILENYA), ketamine, EPI-743 (vatiquinone), sarizotan (EMD-128,130), a statin (e.g. lovastatin), a tricyclic antidepressant (TCA, e.g. desipramine), glatiramer acetate (e.g. COPAXONE), dextromethorphan, and/or an oral cholesterol 24-hydroxylase (CH24H) inhibitor (e.g. TAK-935/OV935).

As stated hereinabove, the instant invention provides nucleic acid molecules, vectors, and compositions and methods for the inhibition, treatment, and/or prevention of Rett syndrome. Compositions comprising at least one nucleic acid described herein are also encompassed by the instant invention. In a particular embodiment, the composition comprises at least one guide RNA or a nucleic acid molecule encoding the guide RNA (e.g., an expression vector) and at least one pharmaceutically acceptable carrier. The composition may further comprise a nucleic acid molecule encoding an RNA editing enzyme linked or fused to an RNA binding domain. In a particular embodiment, all of the nucleic acid molecules are encoded within a single expression vector (e.g., viral vector (e.g., AAV)). Alternatively, the nucleic acid molecules may be contained within separate compositions with at least one pharmaceutically acceptable carrier. The present invention also encompasses kits comprising a first composition comprising at least one guide RNA or a nucleic acid molecule encoding the guide RNA (e.g., an expression vector) and a second composition comprising at least one nucleic acid molecule encoding an RNA editing enzyme linked or fused to an RNA binding domain. The first and second compositions may further comprise at least one pharmaceutically acceptable carrier. In a particular embodiment, the kits of the instant invention comprise a first composition comprising at least one guide RNA or a nucleic acid molecule encoding the guide RNA (e.g., an expression vector) and/or nucleic acid molecule encoding an RNA editing enzyme linked or fused to an RNA binding domain. The first and second compositions may further comprise at least one pharmaceutically acceptable carrier.

As explained hereinabove, the compositions of the instant invention are useful for treating Rett syndrome. A therapeutically effective amount of the composition may be administered to a subject in need thereof. The dosages, methods, and times of administration are readily determinable by persons skilled in the art, given the teachings provided herein.

The components as described herein will generally be administered to a patient as a pharmaceutical preparation. The term "patient" or "subject" as used herein refers to human or animal subjects. The components of the instant invention may be employed therapeutically, under the guidance of a physician for the treatment of the indicated disease or disorder.

The pharmaceutical preparation comprising the components of the invention may be conveniently formulated for administration with an acceptable medium (e.g., pharmaceutically acceptable carrier) such as water, buffered saline, ethanol, polyol (for example, glycerol, propylene glycol, liquid polyethylene glycol and the like), dimethyl sulfoxide (DMSO), oils, detergents, suspending agents or suitable mixtures thereof. The concentration of the agents in the chosen medium may be varied and the medium may be chosen based on the desired route of administration of the pharmaceutical preparation. Except insofar as any conventional media or agent is incompatible with the agents to be administered, its use in the pharmaceutical preparation is contemplated.

Selection of a suitable pharmaceutical preparation depends upon the method of administration chosen. For example, the components of the invention may be administered by direct injection into any desired tissue (e.g., brain) or into the surrounding area. In this instance, a pharmaceutical preparation comprises the components dispersed in a medium that is compatible with blood or the target tissue.

The therapy may be, for example, administered parenterally, by injection into the blood stream (e.g., intravenous), or by subcutaneous, intramuscular or intraperitoneal injection. In a particular embodiment, the therapy is administered by direct injection (e.g., into the tissue to be treated). Pharmaceutical preparations for injection are known in the art. If injection is selected as a method for administering the therapy, steps must be taken to ensure that sufficient amounts of the molecules reach their target cells to exert a biological effect.

Pharmaceutical compositions containing a compound of the present invention as the active ingredient in intimate admixture with a pharmaceutical carrier can be prepared according to conventional pharmaceutical compounding techniques. The carrier may take a wide variety of forms depending on the form of preparation desired for administration, e.g., intravenous, oral or parenteral. In the preparation of an oral dosage form, any of the usual pharmaceutical media may be employed, such as, for example, water, glycols, oils, alcohols, flavoring agents, preservatives, coloring agents and the like in the case of oral liquid preparations (such as, for example, suspensions, elixirs and solutions); or carriers such as starches, sugars, diluents, granulating agents, lubricants, binders, disintegrating agents and the like in the case of oral solid preparations (such as, for example, powders, capsules and tablets). Injectable suspensions may be prepared, in which case appropriate liquid carriers, suspending agents and the like may be employed.

A pharmaceutical preparation of the invention may be formulated in dosage unit form for ease of administration and uniformity of dosage. Dosage unit form, as used herein, refers to a physically discrete unit of the pharmaceutical preparation appropriate for the patient undergoing treatment. Each dosage should contain a quantity of active ingredient calculated to produce the desired effect in association with the selected pharmaceutical carrier. Procedures for determining the appropriate dosage unit are well known to those skilled in the art. Dosage units may be proportionately increased or decreased based on the weight of the patient. Appropriate concentrations for alleviation of a particular pathological condition may be determined by dosage concentration curve calculations, as known in the art.

The methods of the instant invention may further comprise monitoring the disease or disorder in the subject after administration of the composition(s) of the instant invention to monitor the efficacy of the method. For example, the subject may be monitored for characteristics of Rett syndrome.

### Definitions

The following definitions are provided to facilitate an understanding of the present invention:
The singular forms "a," "an," and "the" include plural referents unless the context clearly dictates otherwise.

"Pharmaceutically acceptable" indicates approval by a regulatory agency of the Federal or a state government or listed in the U.S. Pharmacopeia or other generally recognized pharmacopeia for use in animals, and more particularly in humans.

A "carrier" refers to, for example, a diluent, adjuvant, preservative (e.g., Thimersol, benzyl alcohol), anti-oxidant (e.g., ascorbic acid, sodium metabisulfite), solubilizer (e.g., Tween^{®} 80, Polysorbate 80), emulsifier, buffer (e.g., Tris HCl, acetate, phosphate), antimicrobial, bulking substance (e.g., lactose, mannitol), excipient, auxiliary agent or vehicle with which an active agent of the present invention is administered. Pharmaceutically acceptable carriers can be sterile liquids, such as water and oils, including those of petroleum, animal, vegetable or synthetic origin. Water or aqueous saline solutions and aqueous dextrose and glycerol solutions are preferably employed as carriers, particularly for injectable solutions. Suitable pharmaceutical carriers are described in Remington: The Science and Practice of Pharmacy, (Lippincott, Williams and Wilkins); Liberman, et al., Eds., Pharmaceutical Dosage Forms, Marcel Decker, New York, N.Y.; and Rowe, et al., Eds., Handbook of Pharmaceutical Excipients, Pharmaceutical Pr.

The term "treat" as used herein refers to any type of treatment that imparts a benefit to a patient afflicted with a disease, including improvement in the condition of the patient (e.g., in one or more symptoms), delay in the progression of the condition, etc.

As used herein, the term "prevent" refers to the prophylactic treatment of a subject who is at risk of developing a condition resulting in a decrease in the probability that the subject will develop the condition.

A "therapeutically effective amount" of a compound or a pharmaceutical composition refers to an amount effective to prevent, inhibit, or treat a particular disorder or disease and/or the symptoms thereof.

As used herein, the term "subject" refers to an animal, particularly a mammal, particularly a human.

The term "isolated" refers to the separation of a compound from other components present during its production. "Isolated" is not meant to exclude artificial or synthetic mixtures with other compounds or materials, or the presence of impurities that do not substantially interfere with the fundamental activity, and that may be present, for example, due to incomplete purification, or the addition of stabilizers.

The terms "linker", "linker domain", and "linkage" refer to a chemical moiety comprising a covalent bond or a chain of atoms that covalently attaches at least two compounds, for example, an RNA editing enzyme and an RNA binding domain. The linker may be an amino acid sequence (e.g., 1-50 amino acids, 1-25 amino acids, 1-20 amino acids, 1-15 amino acids, 1-10 amino acids, or 1-5 amino acids).

The term "oligonucleotide," as used herein, includes a nucleic acid molecule comprised of two or more ribo- and/or deoxyribonucleotides, preferably more than three. The exact size of the oligonucleotide will depend on various factors and on the particular application and use of the oligonucleotide.

"Nucleic acid" or a "nucleic acid molecule" as used herein refers to any DNA or RNA molecule, either single or double stranded and, if single stranded, the molecule of its complementary sequence in either linear or circular form. In discussing nucleic acid molecules, a sequence or structure of a particular nucleic acid molecule may be described herein according to the normal convention of providing the sequence in the 5' to 3' direction. With reference to nucleic acids of the invention, the term "isolated nucleic acid" is sometimes used. This term, when applied to DNA, refers to a DNA molecule that is separated from sequences with which it is immediately contiguous in the naturally occurring genome of the organism in which it originated. For example, an "isolated nucleic acid" may comprise a DNA molecule inserted into a vector, such as a plasmid or virus vector, or integrated into the genomic DNA of a prokaryotic or eukaryotic cell or host organism.

A "vector" is a genetic element, such as a plasmid, cosmid, bacmid, phage or virus, to which another genetic sequence or element (either DNA or RNA) may be attached. The vector may be a replicon so as to bring about the replication of the attached sequence or element. A vector may be either RNA or DNA and may be single or double stranded. A vector may comprise expression operons or elements such as, without limitation, transcriptional and translational control sequences, such as promoters, enhancers, translational start signals, polyadenylation signals, terminators, and the like, and which facilitate the expression of a polynucleotide or a polypeptide coding sequence in a host cell or organism.

An "expression operon" refers to a nucleic acid segment that may possess transcriptional and translational control sequences, such as promoters, enhancers, translational start signals (e.g., ATG or AUG codons), polyadenylation signals, terminators, and the like, and which facilitate the expression of a nucleic acid or a polypeptide coding sequence in a host cell or organism. An "expression vector" is a vector which facilitates the expression of a nucleic acid or a polypeptide coding sequence in a host cell or organism.

As used herein, a "nuclear localization signal" (NLS) refers to a molecule or polypeptide that facilitates the movement of an attached polypeptide to the nucleus of the cell. In a particular embodiment, a nuclear localization signal is a peptide that directs proteins to the nucleus. Typically, an NLS comprises mostly basic, positively charged amino acids (particularly lysines and arginines). NLS may be monopartite, bipartite, or multipartite. NLS are typically short peptides (e.g., less than about 20 amino acids, less than about 15 amino acids, or less than about 10 amino acids). Examples of NLS are provided in Kosugi et al. (J. Biol. Chem. (2009) 284:478-485; incorporated by reference herein). In a particular embodiment, the NLS comprises the consensus sequence K(K/R)X(K/R) (e.g., a monopartite NLS). In a particular embodiment, the NLS comprises the consensus sequence (K/R)(K/R)X₁₀₋₁₂(K/R)_{3/5}, where (K/R)_{3/5} represents at least three of the five amino acids is either lysine or arginine. In a particular embodiment, the NLS is the SV40 Large T-antigen NLS (e.g., PKKKRKV (SEQ ID NO: 47)). In a particular embodiment, the c-myc NLS comprises the sequence PAAKRVKLD (SEQ ID NO: 54). In a particular embodiment, the NLS is the nucleoplasmin NLS KRPAATKKAGQAKKKK (SEQ ID NO: 60). With regard to the provided sequences, the lysine and arginine amino acids are interchangeable.

In various embodiments, the inclusion of an NLS reduces or abrogates off-target editing, e.g. relative to editing in the absence of an NLS. For instance, in various embodiments, the present gene editing methods involving an NLS reduce off target editing by about 10%, or about 20%, or about 30%, or about 40%, or about 50%, or about 60%, or about 70%, or about 80%, or about 90%, or about 100%. In various embodiments, the present gene editing methods involving an NLS reduce off target editing by about 2-, or about 3-, or about 5-, or about 10-, or about 30-fold.

Additional aspects and embodiments are set out in the numbered paragraphs below, which form part of the description.
1. A method for editing a target sequence of an endogenous RNA in a cell, said method comprising delivering to the cell:
   a) a nucleic acid molecule encoding a fusion protein comprising an RNA editing enzyme linked to an RNA binding domain, and
   b) a nucleic acid molecule encoding a guide RNA,
      wherein said fusion protein comprises a nuclear localization signal,
      wherein said guide RNA comprises a sequence specifically recognized by said RNA binding domain, and
      wherein said guide RNA specifically hybridizes with the target sequence in said endogenous RNA and comprises a mismatch at a nucleotide to be edited.
2. The method of paragraph 1, wherein said endogenous RNA is within the nucleus of said cell.
3. The method of paragraph 1, wherein said RNA editing enzyme is a deaminase.
4. The method of paragraph 1, wherein said RNA editing enzyme is an Adenosine Deaminase Acting on RNA (ADAR).
5. The method of paragraph 4, wherein said ADAR is selected from the group consisting of ADAR1, ADAR2, fragments thereof, and variants thereof.
6. The method of paragraph 5, wherein said ADAR comprises at least 90% identity with SEQ ID NO: 55.
7. The method of any one of paragraphs 1-6, wherein said RNA binding domain is the λN peptide or a variant thereof.
8. The method of paragraph 7, wherein said RNA binding domain comprises at least 90% identity with SEQ ID NO: 46.
9. The method of any one of paragraphs 1-8, wherein said sequence specifically recognized by said RNA binding domain is the BoxB sequence.
10. The method of any one of paragraphs 1-9, wherein said endogenous RNA is an RNA which is expressed in a central nervous system cell.
11. The method of paragraph 10, wherein said endogenous RNA is methyl CpG binding protein 2 (MECP2) RNA.
12. The method of any one of paragraphs 1-11, wherein said guide RNA further comprises one or more mismatches upstream or downstream of the nucleotide to be edited.
13. The method of any one of paragraphs 1-12, wherein said nuclear localization signal is the SV40 Large T-antigen nuclear localization signal or a variant thereof.
14. The method of paragraph 13, wherein said nuclear localization signal comprises SEQ ID NO: 47 or SEQ ID NO: 47 with one, two, or three substitutions, additions, or deletions.
15. The method of paragraph 1, wherein said nuclear localization signal is the SV40 Large T-antigen nuclear localization signal, wherein said RNA binding domain is the λN peptide, wherein said RNA editing enzyme is an Adenosine Deaminase Acting on RNA (ADAR), and wherein said sequence specifically recognized by said RNA binding domain is the BoxB sequence.
16. The method of any one of paragraphs 1-15, wherein the nucleic acid molecules of a) and b) are contained within a single vector.
17. The method of paragraph 16, wherein said vector is a viral vector.
18. The method of paragraph 17, wherein said viral vector is an adeno-associated virus (AAV).
19. A method for editing a target sequence of an endogenous RNA in a cell, said method comprising delivering to the cell a nucleic acid molecule encoding a guide RNA,
   wherein said guide RNA comprises a sequence specifically recognized by an endogenous human Adenosine Deaminase Acting on RNA (ADAR), and
   wherein said guide RNA specifically hybridizes with the target sequence in said endogenous RNA and comprises a mismatch at a nucleotide to be edited.
20. The method of paragraph 19, wherein said endogenous RNA is within the nucleus of said cell.
21. The method of paragraph 19, wherein said ADAR is ADAR1 or ADAR2.
22. The method of paragraph 21, wherein said ADAR comprises at least 90% identity with SEQ ID NO: 55.
23. The method of paragraph 19, wherein said endogenous RNA is methyl CpG binding protein 2 (MECP2) RNA.
24. The method of any one of paragraphs 19-23, wherein said guide RNA further comprises one or more mismatches upstream or downstream of the nucleotide to be edited.
25. The method of any one of paragraphs 19-24, wherein the endogenous ADAR recognizes the guide RNA.
26. The method of any one of paragraphs 19-25, wherein the endogenous ADAR deaminates the base of a nucleotide in the endogenous RNA.
27. The method of any one of paragraphs 19-26, wherein the editing of the target sequence alters levels and/or function of a protein encoded by the target sequence.
28. The method of any one of paragraphs 19-27, wherein the nucleic acid molecule is contained within a viral vector.
29. The method of paragraph 28, wherein said viral vector is an adeno-associated virus (AAV).
30. A method of treating, inhibiting, and/or preventing a genetic disease of the central nervous system in a subject, said method comprising administering to said subject:
   a) a nucleic acid molecule encoding a fusion protein comprising an RNA editing enzyme linked to an RNA binding domain, and
   b) a nucleic acid molecule encoding a guide RNA,
      wherein said fusion protein comprises a nuclear localization signal,
      wherein said guide RNA comprises a sequence specifically recognized by said RNA binding domain, and
      wherein the RNA editing enzyme corrects a mismatch mutation associated with the genetic disease of the central nervous system.
31. The method of paragraph 30, wherein the genetic disease of the central nervous system is Rett syndrome.
32. The method of paragraph 31, wherein the guide RNA specifically hybridizes with methyl CpG binding protein 2 (MECP2) RNA and comprises a mismatch at the mutant nucleotide of the endogenous *MECP2* RNA.
33. The method of any one of paragraphs 30-32, wherein said endogenous RNA is within the nucleus of said cell.
34. The method of any one of paragraphs 30-33, wherein said RNA editing enzyme is an Adenosine Deaminase Acting on RNA (ADAR).
35. The method of paragraph 34, wherein said ADAR is selected from ADAR1, ADAR2, and fragments or variants thereof.
36. The method of paragraph 35, wherein said ADAR comprises at least 90% identity with SEQ ID NO: 55.
37. The method of any one of paragraphs 30-36, wherein said RNA binding domain is the λN peptide or a variant thereof.
38. The method of paragraph 37, wherein said RNA binding domain comprises at least 90% identity with SEQ ID NO: 46.
39. The method of any one of paragraphs 30-38, wherein said sequence specifically recognized by said RNA binding domain is the BoxB sequence.
40. The method of any one of paragraphs 30-39, wherein said nuclear localization signal is the SV40 Large T-antigen nuclear localization signal or a variant thereof.
41. The method of paragraph 40, wherein said nuclear localization signal comprises SEQ ID NO: 47 or SEQ ID NO: 47 with one, two, or three substitutions, additions, or deletions.
42. The method of paragraph 30, wherein said nuclear localization signal is the SV40 Large T-antigen nuclear localization signal, wherein said RNA binding domain is the λN peptide, wherein said RNA editing enzyme is an Adenosine Deaminase Acting on RNA (ADAR), and wherein said sequence specifically recognized by said RNA binding domain is the BoxB sequence.
43. The method of any one of paragraphs 30-42, wherein the nucleic acid molecules of a) and b) are contained within a single vector.
44. The method of paragraph 43, wherein said vector is a viral vector.
45. The method of paragraph 44, wherein said viral vector is an adeno-associated virus (AAV).
46. A method of treating, inhibiting, and/or preventing a genetic disease of the central nervous system in a subject, said method comprising administering to said subject a nucleic acid molecule encoding a guide RNA, wherein said guide RNA comprises a sequence specifically recognized by an endogenous human Adenosine Deaminase Acting on RNA (ADAR).
47. The method of paragraph 46, wherein the genetic disease of the central nervous system is Rett syndrome.
48. The method of paragraph 47, wherein the guide RNA specifically hybridizes with methyl CpG binding protein 2 (MECP2) RNA and comprises a mismatch at the mutant nucleotide of the endogenous *MECP2* RNA.
49. The method of any one of paragraphs 46-48, wherein the endogenous RNA is within the nucleus of said cell.
50. The method of any one of paragraphs 46-49, wherein said ADAR is ADAR1 or ADAR2 or a variant thereof.
51. The method of paragraph 50, wherein said ADAR comprises at least 90% identity with SEQ ID NO: 55.
52. The method of any one of paragraphs 46-51, wherein an endogenous ADAR recognizes the guide RNA.
53. The method of any one of paragraphs 46-52, wherein an endogenous ADAR deaminates the base of a nucleotide in the endogenous RNA.
54. The method of any one of paragraphs 46-53, wherein the editing of the target sequence alters levels and/or function of a protein encoded by the target sequence.
55. The method of any one of paragraphs 46-54, wherein the nucleic acid molecule is contained within a viral vector.
56. The method of paragraph 55, wherein said viral vector is an adeno-associated virus (AAV).

The following examples are provided to illustrate various embodiments of the present invention. The examples are illustrative and are not intended to limit the invention in any way.

### EXAMPLE 1

### Materials and Methods

### Plasmid Constructions

A pcDNA 3.1+ plasmid (Thermo Fisher Scientific) coding for the λN peptide fused to the wild-type ADAR2 catalytic domain was obtained (Montiel-Gonzalez, et al. (2016) Nucleic Acids Res., 44:e157; Montiel-Gonzalez, et al. (2013) Proc. Natl. Acad. Sci., 110:18285-18290). The Editase^{E488Q} cDNA was generated by overlapping PCR of wild-type Editase and cloned into pcDNA3.1+. Both versions of Editase were modified in pcDNA3.1+ by inserting two copies of an HA epitope and three copies of the SV40 NLS in frame and N-terminal to the hybrid Editase (pGM1090, wild type; pGM1091, E488Q). For *Mecp2-BoxB* guides, synthetic oligonucleotides representing the three different *Mecp2* G > A mutations, and their antisense sequences, were annealed with Bsa1 overhangs and cloned into the pENTR/U6 poly linker [pGM1099 (W104X), pGM1181 (306H), pGM1085 (R106Q)] (Thermo Fisher Scientific). The *Mecp2-BoxB* guide containing the off-target A-G mismatch (pGM11089) is also in pENTR/U6. For the *Mecp2* editing substrate, an EcoRI-KpnI fragment of mouse *Mecp2* E1 isoform cDNA (GenBank Accession No. NP_001075448.1) was cloned into the multiple cloning site of pEGFP-N3 (Clontech). Individual G > A mutations of *Mecp2* were generated by overlapping PCR with the same restriction site overhangs and cloned in-frame as a fusion protein with eGFP in pEGFP-N3 (Thermo Fisher Scientific). All subcloning was verified by sequence analysis. Primer sequences used in plasmid constructions and PCR amplifications are found in Table 1.

**Table 1: Guide, PCR and sequencing primers. Provided sequences are SEQ ID NOs: are provided in parentheses.**

| **cDNA and RNA templates** | **Sequence, 5'→3'** |
|---|---|
| **Cloning Mecp2 target plasmids** | |
| mMecp2 E1 Fwd EcoRI | gcgcgaattccaccatggccgccgctgccgccac (1) |
| mMecp2 E1 Rev KpnI no stop codon | gcgcgggtaccgctaactctctcggtcacgggc (2) |
| mMecp2 W104X mut primer Fwd | caccttgcctgaaggttagacacgaaagcttaaac (3) |
| mMecp2 W104X mut primer Rev | gtttaagctttcgtgtctaaccttcaggcaaggtg (4) |
| mMecp2 R106Q mut primer Fwd | cctgaaggttggacacaaaagcttaaacaaagg (5) |
| mMecp2 R106Q mut primer Rev | cctttgtttaagcttttgtgtccaaccttcagg (6) |
| mMecp2 R306H mut primer Fwd | cccatcaagaagcacaagacccgggag (7) |
| mMecp2 R306H mut primer Rev | ctcccgggtcttgtgcttcttgatggg (8) |
| | |

| **Cloning Editase plasmids (pGM1090, pGM1091)** | |
|---|---|
| Editase EcoRI Kozak HA Fwd | gaattcgccaccatggtgtacccctatgacgtgcctgactacgccagcg gctatccatacgatgtccccgattatgctt (9) |
| Editase HA BspEI Rev | ccggaagcataatcggggacatcgtatggatagccgcaggcgtagtca ggcacgtcataggggtaccatggtggcg (10) |
| EGFP F2 | caccatcttcttcaaggacgac (11) |
| SV40 3xNLS Rev | gacaatccggaggtggatcctacctttctctt (12) |
| hDD E488Q Fwd | aatagagtctggtcaggggacgattcc (13) |
| hDD E488Q Rev | ggaatcgtcccctgaccagactctatt (14) |
| | |

| **Guide RNA sequences** | |
|---|---|
| mMecp2 W104X 2xBoxB Guide Fwd | caccgtcctttgtttggccctgaaaaagggccctttcgtgtccaaccttca ggcaaggggccctgaaaaagggccggtcatcatac (15) |
| mMecp2 W104X 2xBoxB Guide Rev | aaaagtatgatgaccggccctttttcagggccccttgcctgaaggttgga cacgaaagggccctttttcagggccaaacaaaggac (16) |
| mMecp2 R106Q 2xBoxB Guide Fwd | caccgcagacttcctggccctgaaaaagggcctttaagctttcgtgtcca accttcaggcaggccctgaaaaagggcctggggtcatc (17) |
| mMecp2 R106Q 2xBoxB Guide Rev | aaaagatgaccccaggccctttttcagggcctgcctgaaggttggacac gaaagcttaaaggccctttttcagggccaggaagtctgc (18) |
| mMecp2 R306H 2xBoxB Guide Fwd | caccgatgctgaccgtggccctgaaaaagggccccgggtcttgcgctt cttgatgggagcaggccctgaaaaagggcctctcatgcaca (19) |
| mMecp2 R306H 2xBoxB Guide Rev | aaaatgtgcatgagaggccctttttcagggcctgctccccatcaagaag cgcaagacccggggccctttttcagggccacggtcagcatc (20) |
| mMecp2 R106Q Off-target mismatch 2xBoxB Guide Fwd | caccgcagacttcctggccctgaaaaagggcctttaagctttccgggtc caaccttcaggcaggccctgaaaaagggcctggggtcatc (21) |
| mMecp2 R106Q Off-target mismatch 2xBoxB Guide Rev | aaaagatgaccccaggccctttttcagggcctgcctgaaggttggaccc gaaagcttaaaggccctttttcagggccaggaagtctgc (22) |
| | |

| **Amplification of Mecp2-eGFP cDNA** | |
|---|---|
| CMV-mMecp2 ATG Fwd | tcaagcttcgaattccaccatggcc (23) |
| GFP-N3 Linker Rev | ccttgctcaccatggtggcga (24) |
| | |

| **Amplification of endogenous Mecp2 cDNA** | |
|---|---|
| mMecp2 -14 mMecp2 ATG Fwd | aacccgtccggaaaatggcc (25) |
| mMecp2-3'UTR+92 Rev | ggaagctttgtcagagccctacccataag (26) |
| | |

| **Sequencing primers for Mecp2 RT-PCR** | |
|---|---|
| mMecp2 554 Rev | ctcctggaggggctccctctc (27) |
| mMecp2 914 Rev | gaccgtatggaagactccttca (28) |
| mMecp2 1122 Rev | actgctgctgcgcccctt (29) |
| | |

| **Cloning of plasmids pGM1257 and pGM1258** | |
|---|---|
| hU6 AflII Fwd | gtgtcttaaggagggcctatttcccatgatt (30) |
| hU6 MfeI Fwd | gtgtcaattggagggcctatttcccatgatt (31) |
| hU6 NheI Fwd | gtgtgctagcgagggcctatttcccatgatt (32) |
| hU6 SacI Fwd | gtgtgagctc gagggcctatttcccatgatt (33) |
| hU6 SpeI Fwd | gtgtactagtgagggcctatttcccatg (34) |
| hU6 (m) NdeI Fwd | gtgtcatatgcttaccgtaacttgaaag (35) |
| R106QgV2 AflII Rev | atatcttaagaaaaaagatgaccccaggccct (36) |
| R106QgV2 Apal Rev | cacagggcccaaaaaagatgaccccaggccct (37) |
| R106QgV2 MfeI Rev | atatcaattgaaaaaagatgaccccaggccct (38) |
| R106QgV2 SacI Rev | atatgagctcaaaaaagatgaccccaggccct (39) |
| R106QgV2 SpeI Rev | atatactagtaaaaaagatgaccccaggccct (40) |
| R106QgV2 (+2)ApaI Rev | gcgcgggcccttcgaagctagcaaaaaagatgaccccaggccct (41) |
| | |

| **Cloning of Editase into plasmid pGM1257** | |
|---|---|
| 2xHA Editase Fwd NcoI Kozak | attcgccaccatggtgtacccctatgacgtg (42) |
| Editase EcoRI Rev | gcgcgaattctcaatggtgatggtgatggt (43) |

### Plasmid Constructs

The initial construct containing the λN peptide and wild-type ADAR2 catalytic domain fusion cDNA (Editase) has been described (Montiel-Gonzalez, et al. (2016) Nucleic Acids Res., 44:e157; Montiel-Gonzalez, et al. (2013) Proc. Natl. Acad. Sci., 110:18285-18290). It was modified to contain two copies of the HA epitope tag followed by three copies of the SV40 NLS in frame and N-terminal to the hybrid Editase (pGM1090). To do this, two single-stranded oligonucleotides encoding two copies of the HA epitope tag and a Kozak sequence were annealed with EcoRI and BspeI overhangs and ligated 5' to the λN domain sequence. Three copies of the SV40 NLS were amplified by PCR from pECFP-Nuc (Clontech) and added between the HA epitope tags and the λN domain using BspEI overhangs. The plasmid pGM1091, which contains the E488Q mutation in the ADAR2 catalytic domain, was generated using the same steps.

Plasmid pGM1258, used for the AAV transduction experiments, contains Editase cDNA under control of the *Synapsin* 1 promoter and six copies of *Mecp2^{R106Q}* guide DNA with the off-target A-G mismatch, each under control of the human U6 promoter. To introduce the *U6-Mecp2^{R106Q}* guide region, the human U6 promoter and CRISPR sgRNA sequences from plasmid pX552 (60958; Addgene; Swiech, et al. (2015) Nat. Biotechnol., 33:102-106) were removed by restriction digest (NdeI/ApaI), and six *U6-Mecp2^{R106Q}* guide sequences were inserted between these sites, in two steps. In the first step, three copies of the *U6-Mecp2^{R106Q}* guide region were cloned into pX552 by a four-way ligation of PCR amplicons (pGM1108 template) with the following restriction sites: NdeI/MfeI, MfeI/SpeI, and SpeI/NheI+ApaI (pGM1257). In the second step, three additional copies of the *U6-Mecp2^{R106Q}* guide region were generated by PCR amplification from pGM1108 using primers adding the following restriction sites: NheI/SacI, SacI/AfIII, and AfIII/ApaI. The final plasmid, pGM1258, was generated by restriction of pGM1257 digested with NheI/ApaI and a four-way ligation with the three *U6-Mecp2^{R106Q}* PCR amplicons. To introduce Editase cDNA into pGM1258, the sequences corresponding to the ORF of Editase were amplified from plasmid pGM1091 and added downstream from the *Synapsin* 1 promoter in pGM1257, using NcoI and EcoRI overhangs.

### AA V Vector and Virus Preparation

The AAV1/2 backbone vector, pX552, containing the human *Synapsin I* promoter was obtained from Addgene (plasmid 60958; Swiech, et al. (2015) Nat. Biotechnol., 33:102-106). pX552 was modified by replacing the eGFP-KASH coding sequence with the HA-tagged NLS Editase cDNA, without and with six copies of guide cDNAs (pGM1186, Editase only; pGM1258, Editase and R106Q guides). Editase and guide sequences were verified by sequence analysis before generating virus.

Each AAV1/2 chimeric vector was produced in human embryonic kidney 293 (HEK293) cells on a scale of three 225 cm² flasks per vector by an adenovirus-free plasmid transfection method (Matsushita, et al. (1998) Gene Ther., 5:938-945; Earley, et al. (2017) J. Virol., 91:e01980-16). In each flask, ~2 × 10⁷ HEK293 cells were transfected with a total of 45 µg of the following four plasmid DNAs mixed with polyethyleneimine (PEI) at a DNA:PEI weight ratio of 1:2. The plasmid DNA mixture contained 15 µg of pHelper (Agilent), 7.5 µg each of pHLP19-1 and pHLP19-2, and one of the AAV vector Editase recombinant plasmids (15 µg) containing AAV vector genome sequences with two inverted terminal repeats (ITRs). pHLP19-1 is an AAV1 helper plasmid supplying AAV2 Rep proteins and AAV1 VP proteins, and pHLP19-2 is an AAV2 helper plasmid supplying AAV2 Rep proteins and AAV2 VP proteins (Grimm, et al. (2003) Blood 102:2412-2419). Three days post-transfection, cells were harvested. AAV vector particles were then recovered from the cells by cell lysis and purified using HiTrap^{™} heparin column (GE Healthcare; Desterro, et al. (2003) J. Cell Sci., 116:1805-1818). The titer of each virus was determined by a quantitative dot blot assay using a probe generated against the Editase coding sequence.

### Cell Culture

Neuro2A cells (ATCC CCL-131) were maintained in DMEM (Thermo Fisher Technologies) in 10% FBS (lot no. AAC20-0955; HyClone) at 37°C in 5% CO₂ humidified incubator. Primary neurons were derived from the *Mecp2^{R106Q}* mouse line that was generated by targeted homologous recombination (Janelia Farms) and characterized by genotyping. All animal studies were approved by the Oregon Health and Science University Institutional Animal Care and Use Committee. Pups (P0) were killed by decapitation and the brains dissected in ice-cold Hanks Basal Salt Solution (HBSS, pH 7.4) with 25 mM Hepes. Individual hippocampi were excised without the meninges and pooled by genotype. The tissue was treated with 1% trypsin and 0.01% DNase I in HBSS at 37°C for 10 minutes. Tissue pieces were rinsed three times at room temperature in HBSS and dissociated in Minimal Essential Media (Gibco) containing 25 mM glucose, 1% pen/strep, 1% horse serum (lot no. B02307-7021; HyClone), and 1% FBS. Neurons were dissociated by filtering through a 0.4-µm filter and plated in poly-L-lysine-coated dishes at a density of 5 × 10⁵ cells per well in a 12-well dish or 5 × 10⁴ in a 96-well glass chamber, in neuronal growth media consisting of Neurobasal-A (Thermo Fisher Scientific), 1× Glutamax (Thermo Fisher Scientific), 2% B27 (Thermo Fisher Scientific), and penicillin/streptomycin. After 24 hours, neurons received a full medium change to remove cellular debris. Half medium changes were done every 2-3 days. Cells were maintained at 37°C in 5% CO₂.

### Generation and Genotyping of Mecp2^{R106Q} Mice

The targeting vector to create the *Mecp2^{R106Q}* mice consisted of *Mecp2* exon 3, followed by a flippase recognition target (frt) flanked neomycin cassette in intron 3, the first 1.2 kb of *Mecp2* exon 4, and the neomycin resistance gene expressed from the phosphoglycerate kinase promoter (PGK). The linearized construct was electroporated into mouse embryonic stem cells (mESCs), and correctly targeted clones were identified by G418 sensitivity and sequencing. Mice expressing the knocked-in *Mecp2^{R106Q}* allele were generated from mESCs by standard procedures. The neomycin resistance cassette was removed by crossing *Mecp2^{R106Q}* mice and mice expressing the flippase recombinase from the Rosa 26 locus (stock no. 009086; Jackson Labs). Removal of the cassette was confirmed by sequencing.

Genotyping of the Mecp2R106Q mice was performed using the following primers: Mecp2-R106Q Fwd (5' ggacctatgtatgatgaccc 3' (SEQ ID NO: 50)) and Mecp2-R106Q Rev (5' ggtcattgggctagactgaa 3' (SEQ ID NO: 51)), which amplify a region of the third intron of the *Mecp2* gene. The amplicon from *Mecp2^{R106Q}* knock-in animals contains the remaining frt site used to remove the neomycin cassette, resulting in a PCR product 93 base pairs larger than the wild type (392 bp vs. 299 bp).

### RNA Editing

For analysis of N2A cells, cells were seeded at a density of 1.3 × 10³ cells per well in a 12-well plate. After 24 hours, cells were transfected with plasmids containing wild-type or E488Q Editase (pGM1090 and 1091), one copy of guide (pGM1099, pGM1181 orpGM1108), and *Mecp2-egfp* cDNAs (pGM1174, pGM1172, or pGM1173) using a 2:1 ratio of Lipofectamine^{™} 2000 (Thermo Fisher Scientific) and DNA in Opti-MEM^{™} reduced serum media (Thermo Fisher Scientific). The amount of plasmid DNA added per well was 125 ng target, 250 ng Editase, and 2.5 µg guide. After 72 hours, cells were harvested and total RNA was isolated using the Purelink^{®} RNA Mini kit (Ambion) according to the manufacturer's instructions. Residual plasmid DNA was removed using the TURBO DNA-free^{™} kit (Ambion). Total RNA was reverse transcribed using the SuperScript^{®} III First-Strand Synthesis System (Life Technologies) and primed using oligo dT. The transfected *Mecp2-egfp* cDNAs were amplified for sequence analysis by PCR using a 5' primer in the CMV promoter in pEGFP-N3 and a reverse primer in the egfp gene. For editing analysis of primary neurons, at DIV7, 5 × 10⁵ hippocampal primary neurons were transduced with AAV1/2 at a multiplicity of infection of 3-6 × 10⁴ viral genomes per cell. Viral volume did not exceed 5% of total medium volume. Cells were harvested 1-week post-transduction and analyzed for editing efficiency as described for the transfected N2A cells.

The efficiency of A to I editing was determined by reverse transcription PCR (RT-PCR) and direct sequencing of PCR products. Quantification of the sequencing peak heights from the antisense strand was determined by processing the four-dye-trace sequences using the Bioedit Software package (mbio.ncsu.edulBioEdit/ bioedit.html; File > Batch Export of Raw Sequence Trace Data). The amount of editing at each site was then determined using the maximum height of the T (nonedited) and C (edited) peaks at a given site and calculating the percentage of cDNA edited {100% × [C height/(T height + C height)]}. A detection limit of 5% editing was determined by measuring G-A peak heights in mixtures containing decreasing ratios of R106Q mutant to wild-type Mecp2 plasmids. The C/T peak heights of the antisense strand were quantified because it is more accurate than using the A/G peak heights of the sense strand (Eggington, et al. (2011) Nat. Commun., 2:319). However, for clarity, all chromatograms are shown in the reverse complement.

### Western Blotting

Primary hippocampal neurons, transduced with AAV1/2, were lysed in 100 µL of whole-cell lysis buffer (25 mM Tris, pH 7.6, 150 mM NaCl, 1% Igepal CA-630; Sigma), 1% deoxycholate, 0.1% SDS, protease inhibitor (Complete EDTA-free; Roche), 1 mM beta-mercaptoethanol, and 250 units per mL benzonase (Sigma-Aldrich). Lysates were centrifuged at 9,300 × g for 10 minutes at 4°C and the soluble fraction isolated. Protein concentrations were measured using the BCA protein assay kit (Pierce Biotechnology). Equal amounts of protein lysates were separated on NuPage^{®} 4-12% Bis-Tris gels (Thermo Fisher Scientific) in Mops-SDS running buffer (Thermo Fisher Scientific), and proteins were blotted onto a nitrocellulose membrane (GE Healthcare Life Sciences). Membranes were blocked with 3% BSA in 1× TBST (TBS with 0.05% Tween^{®} 20) for 1 hour, then incubated with either rabbit anti-mMeCP2 (Covance) or rabbit anti-β-actin (8227; Abcam) overnight at 4°C. After washing three times with 1× TBST, blots were incubated with anti-rabbit IgG DyLight^{®} 680 (1:10,000 dilution; Thermo Scientific) for 1 hour. Blots were quantified using the Odyssey^{®} Imaging System (LI-COR Biosciences).

### Immunostaining

Hippocampal primary neurons were fixed in 4% paraformaldehyde in PBS for 20 minutes at room temperature. Fixed cells were washed twice with 1× PBSG (0.1 M glycine in 1× PBS) at room temperature for 10 minutes. Then, cells were blocked and permeabilized [0.5% Igepal CA-630, Sigma; 3% BSA (source) in 1× PBS] for 1 hour at 4°C and incubated with primary antibodies raised against MeCP2 (rabbit mAb D4F3; Cell Signaling) and HA (rat mAb 3F10; Roche) in a humidified chamber overnight at 4°C. Cells were washed three times in 1× PBS containing 0.5% Igepal and incubated with secondary antibodies Alexa 488 and Alexa 568 (Thermo Fisher Scientific) for 1 hour. After another wash with 1× PBS containing 0.5% Igepal, cells were incubated with 300 nM DAPI for 5 minutes, then washed again with 1× PBS. The cells were mounted using ProLong^{®} Gold antifade reagent (Thermo Fisher Scientific) overnight. All images were acquired as z-stacks of 0.5-pm optical sections on a Zeiss 710 confocal microscope using a 40× water immersion objective. HA and MeCP2 fluorescent images were taken using the same settings across all samples. Total cell number or numbers of antibody-positive cells were determined by ImageJ cell counter plugin (National Institutes of Health, imagej.nih.gov/ij, version 1.60_65 (32 bit)).

### Statistical Analysis

All statistics were performed using GraphPad version 6.0 software (Prism). The percentage of A to I editing in N2A cells was analyzed using one-way ANOVA followed by Bonferroni post hoc tests. The level of A to I editing in *Mecp2*^{*R106Q*/*y*} transduced neurons, Western blots comparing MeCP2 protein levels, and the number of neurons showing MeCP2 enrichment at heterochromatic foci were each analyzed using unpaired t tests. All experimental results are expressed as mean ± SD.

### Results

### Targeting Editase to Heterologously Expressed Mecp2 mRNA Can Repair Mecp2 G > A Mutations

There are at least three G > A mutations in human *MECP2* that give rise to classical Rett syndrome. Two mutations reside within the Methyl DNA Binding Domain (MBD), MeCP2^{R106Q} and MeCP2^{W104X}, and one, MeCP2^{R306H}, resides in the NCoR interaction domain (NID) (Fyfe, et al. (2003) J. Child. Neurol., 18:709-713; Lyst, et al. (2013) Nat. Neurosci., 16:898-902) (Fig. 1A). To determine whether Editase could repair these mutations, editing was tested following transient transfections of Editase, guide RNA, and *Mecp2* cDNAs into N2A cells. To distinguish heterologously expressed MeCP2 proteins from endogenous ones, the heterologously expressed MeCP2 was tagged with C-terminal eGFP. Editase and *Mecp2-gfp* cDNAs were expressed from the cytomegalovirus (CMV) immediate early gene promoter-enhancer, and guide was expressed from the human U6 small nuclear RNA gene promoter. Three copies of the Simian virus 40 large T antigen nuclear localization signal (NLS) were added to the Editase, in addition to the λN peptide, because ADAR2 edits endogenous mRNAs in the nucleus as a primary transcript (Desterro, et al. (2003) J. Cell. Sci., 116:1805-1818). Each guide RNA contains two stem loops (BoxB) representing the sequences recognized by the λN peptide. One BoxB stem loop is located 16-18 bases 5' of the target A, and the second is located 10 bases 3' of the target A (Fig. 1B). The number and position of the stem loops relative to the target A were based on studies (Montiel-González, et al. (2016) Nucleic Acids Res., 44:e157; Montiel-Gonzalez, et al. (2013) Proc. Natl. Acad. Sci., 110:18285-18290) and determined empirically for *Mecp2* in transfection analyses. Editing is optimal with a C mismatch at that site in the complementary guide (Schneider, et al. (2014) Nucleic Acids Res., 42:e87; Wong, et al. (2001) RNA 7:846-858; Källman, et al. (2003) Nucleic Acids Res., 31:4874-4881), and all *Mecp2* guide mRNAs contain this mismatch.

The N2A cells were cotransfected with separate plasmids encoding Editase, MeCP2-GFP, and a third plasmid either containing or lacking the guide sequences. After 3 days, Sanger sequencing was used to analyze cDNAs synthesized from the targeted region of *Mecp2-gfp* mRNA (Figs. 1C and 1D). Editing efficiency was measured by determining relative peak heights at the targeted A position. All three *Mecp2* mutations were edited in a guide-dependent manner, consistent with ADAR2-mediated editing requiring double-stranded RNA (Figs. 1C and 1D). The percent editing for a targeted A varied with the 5' nucleotide context, similar to the sequence preference of the ADAR2 catalytic domain (Eggington, et al. (2011) Nat. Commun., 2:319; Lehmann, et al. (2000) Biochemistry 39:12875-12884). Specifically, based on *in vitro* screens, the optimal 5' nucleotide hierarchy for A deamination by ADAR2 catalytic domain is U > A> C > G and the most optimal 3' nucleotides are C ~ G ~ A > U. W104X (UAG) was edited most efficiently (76 ± 10%), followed by R306H (**C**A**C**, 34 ± 3%) and R106Q (**C**A**A**, 25 ± 2%), which for *Mecp2* were not statistically different (Fig. 1D). To further optimize the Editase system for repairing *Mecp2* G > A mutations, R106Q was focused on because in human patients it is more common than the W104X mutation and leads to a more severe form of Rett syndrome than R306H (Fyfe, et al. (2003) J. Child. Neurol., 18:709-713; Cuddapah, et al. (2014) J. Med. Genet., 51:152-158).

### A Mutation in the Deaminase Domain, E488Q, Increases Editing Efficiency of the Hybrid Editase

hADAR2 catalytic domains containing an E488Q mutation increase A > I editing efficiency by increasing both the catalytic rate (Montiel-González, et al. (2016) Nucleic Acids Res., 44:e157; Kuttan, et al. (2012) Proc. Natl. Acad. Sci., 109:E3295-E3304) and the affinity of the catalytic domain for substrate RNAs (Lehmann, et al. (2000) Biochemistry 39:12875-12884). This feature allows the E488Q mutation to achieve higher editing levels of unfavorable 5' and 3' contexts (Montiel-González, et al. (2016) Nucleic Acids Res., 44:e157; Kuttan, et al. (2012) Proc. Natl. Acad. Sci., 109:E3295-E3304). To test whether Editase^{E488Q} would increase the editing efficiency of the target A in *Mecp2^{R106Q},* which has a suboptimal 5' C, N2A cells were cotransfected with *Mecp2^{R106Q}-egfp* and Editase^{E488Q} cDNAs. Sequence analysis indicated that guide expression was required for editing and that the percent editing of *Mecp2* mRNA was increased -two-fold with Editase^{E488Q} compared with wild-type Editase (51 ± 11% vs. 22 ± 5%, n = 3, P < 0.01) (Fig. 2A).

Using either wild-type hADAR2 or hADAR2^{E488Q} catalytic domains in the hybrid Editase, one off-target editing site was detected within the guide region of transfected *Mecp2^{R106Q}-egfp* cDNA (Fig. 2B). Editing at this site results in a silent codon change, T105T (ACA > ACG). A G mismatch at the off-target site can reduce off-target editing in transfected substrates (Vogel, et al. (2014) Angew Chem. Int. Ed. Engl., 53:6267-6271). To determine whether a G mismatch would also reduce off-target editing in *Mecp2* mRNA, editing efficiency was analyzed in N2A cells transfected with plasmids coding for *Mecp2^{R106Q}-egfp* cDNA, Editase^{E488Q}, and a guide with a G mismatch at the nearby off-target A (Fig. 2C). The amount of off-target editing was reduced significantly when the Editase was targeted with a guide containing the A-G mismatch (4.9 ± 0% with mismatch, 33 ± 5% without mismatch, n = 3, *P* < 0.0001; Figs. 2D and 2E), with no significant effect on editing at the target A (Figs. 2D and 2F). All of the editing events required the presence of the guide RNA (Figs. 2E and 2F).

### Site-Directed RNA Editing Repairs an Endogenous Rett-Causing Mutation, Restoring Protein Levels and MeCP2 Function

Next, it was tested whether Editase^{E488Q} could (i) repair the R106Q missense mutation in the endogenous *Mecp2* mRNA, (ii) recover protein levels, and (iii) restore the ability of MeCP2 to bind to heterochromatin, a hallmark functional feature required to reverse Rett-like symptoms in mice (Garg, et al. (2013) J. Neurosci., 33:13612-13620). For these tests, neurons were isolated from mice and engineered to contain the R106Q mutation in the endogenous *Mecp2* gene. The cultured neurons were transduced with either of two AAVs (AAV1/2). Both viruses expressed Editase^{E488Q} under control of the human *Synapsin I* promoter (Swiech, et al. (2015) Nat. Biotechnol., 33:102-106), and one virus additionally contained six copies of the guide (off-target mismatch guide; Fig. 2C) each under control of the human U6 promoter. The other virus served as a control and lacked all guide sequences. Hippocampal neurons were generated from P0 *Mecp2*^{*R106Q*/*y*} mice and transduced with either guide-containing or control AAV vectors carrying the AAV1/2 hybrid capsids at 7 days *in vitro* (DIV 7). After allowing expression of the virus for an additional 7 days, *Mecp2* cDNA was prepared from experimental and control cultures and analyzed by Sanger sequencing. It was found that 72 ± 5% of the *Mecp2* mRNA was repaired in the cultures expressing both Editase and guide (Fig. 3A), while there was no detectable editing in neurons transduced with the control virus that lacked guide. In addition to editing at R106Q, sequence analysis also identified several off-target editing sites within the *Mecp2* cDNA (Fig. 3B). The off-target sites occurred primarily within the region complementary to the guide RNA, although one event occurred outside the guide (N126S).

The functional consequences of the RNA editing was tested by measuring the amount of MeCP2 protein in the AAV1/2 transduced cultures by Western blotting. Similar to other mutations in the MBD (Goffin, et al. (2011) Nat. Neurosci., 15:274-283; Brown, et al. (2016) Hum. Mol. Genet., 25:558-570), MeCP2^{R106Q} protein levels are decreased compared with wild-type levels (Fig. 4). The reduced levels of mutant MeCP2 protein are likely due to destabilization (Goffin, et al. (2011) Nat. Neurosci., 15:274-283). Expression of the Editase and guide in the mutant primary neurons increased MeCP2 protein levels by -three-fold compared with expression of Editase alone (Fig. 4; 35.3 ± 2% with guide compared with 12.9 ± 1% without guide, *n* = 3, *P* < 0.001). This demonstrates for the first time the functional recovery of an endogenous disease causing protein after editing.

MeCP2 binds with high affinity to methyl-CpGs, both *in vitro* and *in vivo* (Skene, et al. (2010) Mol. Cell., 37:457-468; Lagger, et al. (2017) PLoS Genet., 13: e1006793), a property critical to normal function. In mouse cells, mutations in the MBD of MeCP2 reduce binding to heterochromatin that contains amplified satellite sequences rich in mCG (Brown, et al. (2016) Hum. Mol. Genet., 25:558-570; Heckman, et al. (2014) eLife 3:e02676). MeCP2^{R106Q}, an MBD mutation, also shows reduced binding to methyl-CpGs in vitro (Yang, et al. (2016) ACS Chem. Biol., 11:2706-2715). To determine whether MeCP2^{R106Q} has similarly reduced binding in cells and whether editing of G > A mutant *Mecp2* RNA restores enrichment in heterochromatin, nuclei were immunolabeled in *Mecp2*^{*R106Q*/*y*} neuronal cultures transduced with AAV1/2 encoding HA-tagged Editase, with or without guide as a control (Fig. 5). DAPI (4', 6-diamidino-2-phenylindole), a fluorescent indicator that binds strongly to A-T-rich regions in DNA, was used to identify nuclei and heterochromatin. In cultures from wild-type neurons (*Mecp2*^{*+*/*y*}), nuclei showed classical MeCP2 enrichment in the DAPI-stained heterochromatin (foci), reflecting a functional MBD (Fig. 5A). In contrast, in cultures prepared from *Mecp2*^{*R106Q*/*y*} siblings transduced with Editase virus that lacked guide sequences, MeCP2 immunofluorescence was distributed diffusely throughout the nucleus, as expected for a mutation in the MBD that prevents binding to DNA (Goffin, et al. (2011) Nat. Neurosci., 15:274-283; Heckman, et al. (2014) eLife 3:e02676) (Fig. 5B). The intensity of staining was also less than in wild-type nuclei, presumably reflecting the destabilized MeCP2 protein. In contrast, *Mecp2^{R106Q}* neurons expressing both Editase and guide RNA showed a clear increase in MeCP2 immunofluorescence, to a level similar to wild-type nuclei, and enrichment of MeCP2 protein at heterochromatic foci, indicating functional restoration of the MBD (Figs. 5C and 5D). To quantify the immunofluorescence results, it was first determined in three experiments that Editase was expressed in the same percentage of cells irrespective of the presence of guide (Editase alone 67 ± 7%, Editase and guide 67 ± 10%; n = 134 and 137 cells, respectively; Fig. 5E). It was then determined that in the cultures transduced with Editase and guide, 74 ± 11% of the cells expressing Editase (Fig. 5F) and 49 ± 8% of the total cells showed MeCP2 enrichment in heterochromatic foci (Fig. 5G). Enrichment of MeCP2 was never detected within heterochromatic foci in *Mecp2^{R106Q}* nuclei transduced with virus lacking guide, consistent with the sequencing results showing that editing depended upon the presence of guide.

ADAR has been used to repair G > A mutations in exogenous mRNAs in *Xenopus* oocytes (Woolf, et al. (1995) Proc. Natl. Acad. Sci., 92:8298-8302). The data presented herein demonstrates that site-directed RNA editing, using an engineered hADAR2 catalytic domain, can repair an endogenous mutant mRNA and reverse a cellular defect caused by the mutation.

Three genes encode ADAR proteins in mouse and human, but only ADAR1 and ADAR2 exhibit A-to-I catalytic activity (Nishikura, K. (2010) Annu. Rev. Biochem., 79:321-349). Native ADAR-mediated editing is critically important for post-transcriptionally modulating protein function in the brain, first shown for ion channels and receptors (Bhalla, et al. (2004) Nat. Struct. Mol. Biol., 11:950-956; Sommer, et al. (1991) Cell 67:11-19; Burns, et al. (1997) Nature 387:303-308) but now known to extend to many other proteins and noncoding RNAs (Chen, et al. (2012) Curr. Top. Microbiol. Immunol., 353:111-121; Nishikura, K. (2016) Nat. Rev. Mol. Cell Biol., 17:83-96). The ADAR2 catalytic domain was focused on because of its ability to edit heterologous mRNAs (Vogel, et al. (2014) Angew Chem. Int. Ed. Engl., 53:6267-6271; Schneider, et al. (2014) Nucleic Acids Res., 42:
e87; Montiel-González, et al. (2016) Nucleic Acids Res., 44:e157; Montiel-Gonzalez, et al. (2013) Proc. Natl. Acad. Sci., 110:18285-18290; Wong, et al. (2001) RNA 7:846-858) and because of its well-characterized editing mechanism (Kuttan, et al. (2012) Proc. Natl. Acad. Sci., 109:E3295-E3304; Matthews, et al. (2016)
Nat. Struct. Mol. Biol., 23:426-433). Indeed, increased editing efficiency of *Mecp2* mRNA was found when the Editase contained an E488Q mutation within the catalytic domain (Montiel-González, et al. (2016) Nucleic Acids Res., 44:e157; Kuttan, et al. (2012) Proc. Natl. Acad. Sci., 109:E3295-E3304;
Phelps, et al. (2015) Nucleic Acids Res., 43:1123-1132). The elucidation of the structure of the hADAR2 catalytic domain complexed to double-stranded RNA (Matthews, et al. (2016) Nat. Struct. Mol. Biol., 23:426-433) provides a valuable resource for generating other mutations to further optimize editing efficiency and specificity for MeCP2 and other mutations (Wang, et al. (2016) Nucleic Acids Res., 44:9872-9880). In contrast to previous efforts, all of the constructs here included an NLS, which increases editing efficiency, particularly of endogenous mRNAs, because ADARs normally edit primary transcripts within the nucleus (Wong, et al. (2001) RNA 7:846-858).

In transfected cells, the higher editing efficiency with Editase^{E488Q} at the targeted A also resulted in higher off-target editing at one site within the guide region. The single off-target editing site was attenuated by using a G-A mismatch (Schneider, et al. (2014) Nucleic Acids Res., 42:e87). Notably, the sequencing of five cDNAs representing highly expressed mRNAs, other than the target mRNA, did not indicate off-target editing (Montiel-González, et al. (2016) Nucleic Acids Res., 44:e157). However, and surprisingly, in the instant study with neurons, off-target editing sites were different between transfected and endogenous *Mecp2* mRNA. Specifically, in endogenous repaired *Mecp2* mRNA, several additional off-target editing sites within, and one outside, the guide region were found that were absent from the *Mecp2* mRNA expressed from cDNA (Fig. 3B). The difference in off-target editing sites between transfected and endogenous *Mecp2* mRNAs likely reflects sequence differences that can affect RNA folding and other downstream processing events. Importantly, none of the off-target sites in the endogenous *Mecp2* mRNA are reported to cause Rett syndrome (Fyfe, et al. (2003) J. Child Neurol., 18:709-713). Rett syndrome mouse models can be further used to show that cellular and behavioral symptoms are reversed by restoration of wild-type MeCP2 in symptomatic mice (Guy, et al. (2007) Science 315:1143-1147;
Sinnett, et al. (2017) Mol. Ther. Methods Clin. Dev., 5:106-115; Gadalla, et al. (2017) Mol. Ther. Methods Clin. Dev., 5:180-190; Garg, et al. (2013) J. Neurosci., 33:13612-13620; Gadalla, et al. (2013) Mol. Ther., 21:18-30).

### EXAMPLE 2

Mice with the *Mecp2* mutation *Mecp2^{317G>A}* were used to study the instant methods *in vivo.* The *Mecp2^{317G>A}* mutation yields a MeCP2 with the R106Q amino acid change. Briefly, mice with the *Mecp2* mutation *Mecp2^{317G>A}* were treated with AAV vectors encoding the Editase with the E488Q mutation of the instant invention with or without 6 copies of a guide RNA. An AAV vector with the PHP.B capsid (an AAV9 variant) was used because of its neurotropic properties (Bordeaux et al. (2018) Mol. Ther., 26(3):664-668). 1.1 × 10¹⁰ viral genome equivalents (vge) of the AAV was stereotaxically injected into the hippocampus of the mice. 3-4 weeks after direct viral injection, the mice were sacrificed and MeCP2 function was detected in the brain. As seen in Table 2, efficient targeted RNA editing *in vivo* and recovery of MeCP2 function in the brain was observed. Further, based on RNA sequence analysis, the A to I editing efficiency in dentate granule neurons was determined to be 39% whereas the A to I editing efficiency in CA1 neurons was determined to be 64%. As seen in Figure 6, MeCP2 intensity in dentate heterochromatin was greater in mice injected with AAV having the guide RNA in comparison to mice injected with AAV without the guide RNA. These results indicate the rescue of MeCP2 DNA binding ability.

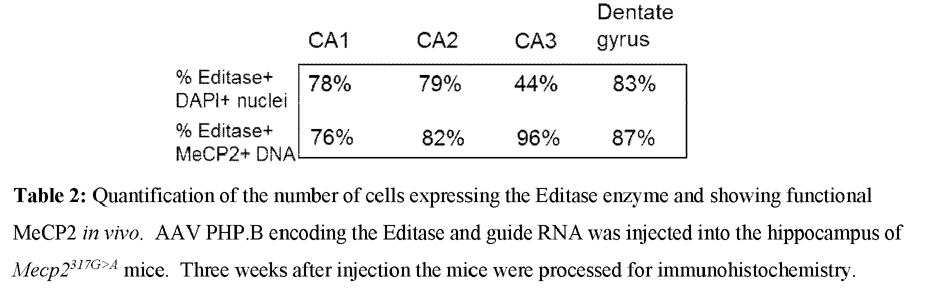

Editase+ cells were identified by HA immunostaining of brain sections of injected mice after thresholding signal from uninfected cells. The percentages are relative to the total number of cells identified by DAPI. The percentage of Editase+ cells showing MeCP2 enrichment within heterochromatin (foci) is indicative of restoration of MeCP2 protein function. n = 864 cells.

### EXAMPLE 3

### Plasmid Constructions

The sequence encoding full-length human ADAR2 containing an amino-terminal Flag tag was subcloned from a yeast expression vector into pcDNA 3.1+ (Thermo Fisher Scientific). To express *Mecp2* guides designed to recruit full length ADAR2, synthetic oligonucleotides were annealed with Bsa1 overhangs and cloned into the pENTR/U6 polylinker [pGM1099 (2xBoxB guide W104X), pGM1192 (internal loop guide W104X), pGM1310 (GluA2 stem loop W104X] (Thermo Fisher Scientific). The *Mecp2* editing substrate containing the *Mecp2^{311G>A}* (W104X) mutation is described in Example 1. All subcloning was verified by sequence analysis. Primer sequences used in plasmid constructions and PCR amplifications are found in Table 3.

### Cell Culture

HEK293T cells (ATCC CRL-3216) were maintained in DMEM (Thermo Fisher Technologies) in 10% FBS at 37°C in 5% CO₂ humidified incubator.

### RNA Editing

For analysis of editing using full length ADAR2, HEK293T cells were seeded at a density of 1.3 × 10³ cells per well in a 12-well plate. After 24 hours, cells were transfected with plasmids encoding the full-length human ADAR2 (pGM1155), one copy of guide (pGM1099, pGM1192, or pGM1310), and *Mecp2^{311G>A}-egfp* cDNA using a 2:1 ratio of Lipofectamine^{™} 2000 (Thermo Fisher Scientific) and DNA in Opti-MEM^{™} reduced serum media (Thermo Fisher Scientific). The amount of plasmid DNA added per well was 125 ng target, 250 ng human ADAR2, and 2.5 µg guide. After 72 hours, cells were harvested and total RNA was isolated using the Purelink^{®} RNA Mini kit (Ambion) according to the manufacturer's instructions. Residual plasmid DNA was removed using the TURBO DNA-free^{™} kit (Ambion). Total RNA was reverse transcribed using the SuperScript^{®} III First-Strand Synthesis System (Life Technologies) and primed using oligo dT. The transfected *Mecp2^{311G>A}-egfp* cDNA was amplified for sequence analysis by PCR using a 5' primer in the CMV promoter in pEGFP-N3 and a reverse primer in the egfp gene.

**Table 3: Guide, PCR and sequencing primers. SEQ ID NOs: are provided in parentheses.**

| **cDNA and RNA templates** | **Sequence, 5'→ 3'** |
|---|---|
| **Cloning hADAR2 plasmid** | |
| hADAR2 3xFlag Fwd | tggtggaattcgccaccatggactacaagg (61) |
| hADAR2 Rev | tcgagcggccgctcaatggtgatggtga (62) |

| **Guide RNA sequences** | |
|---|---|
| mMecp2 W104X 2xBoxB Guide Fwd | caccgtcctttgtttggccctgaaaaagggccctttcgtgtccaaccttca |
| | ggcaaggggccctgaaaaagggccggtcatcatac (15) |
| mMecp2 W104X 2xBoxB Guide Rev | aaaagtatgatgaccggccctttttcagggccccttgcctgaaggttgga cacgaaagggccctttttcagggccaaacaaaggac (16) |
| mMecp2 W104X Internal loop Guide Fwd | caccgacttcctttgttattgctttcgggtccaaccttcaggcatcctgggg tcatcata (63) |
| mMecp2 W104X Internal loop Guide Rev | aaaatatgatgaccccaggatgcctgaaggttggacccgaaagcaata acaaaggaagtc (64) |
| mMecp2 W104X GluA2 Guide Fwd | caccgtggaatagtataacaatatgctaaatgttgttatagtatcccactcg tgtccaaccttcatctagagggccctgaagagggcccttt (65) |
| mMecp2 W104X GluA2 Guide Rev | aaaaaaagggccctcttcagggccctctagatgaaggttggacacgag tgggatactataacaacatttagcatattgttatactattccac (66) |
| | |

| **Amplification of Mecp2-eGFP cDNA** | |
|---|---|
| CMV-mMecp2 ATG Fwd | tcaagcttcgaattccaccatggcc (23) |
| GFP-N3 Linker Rev | ccttgctcaccatggtggcga (24) |
| | |

| **Sequencing primers for Mecp2 RT-PCR** | |
|---|---|
| mMecp2 554 Rev | ctcctggaggggctccctctc (27) |
| mMecp2 914 Rev | gaccgtatggaagactccttca (28) |
| mMecp2 1122 Rev | actgctgctgcgcccctt (29) |

### Results

Human embryonic kidney (HEK) cells were transfected with full length human ADAR2 and *Mecp2^{317G>A}* under control of the cytomegalovirus (CMV) promoter. The human ADAR2 was a full-length native ADAR2 molecule mimicking the endogenous ADAR2. The *Mecp2^{317G>A}* mutation results in an R106Q amino acid change (Mecp2^{R106Q}). The cells were then treated with 1) a guide RNA with 2 BoxB stem loops as described above (see, e.g., Example 1), 2) a guide RNA comprising a R/G binding site from GluA2 (Wettengel, et al. (2017) Nucleic Acids Res., 45(5): 2797-2808; Fukuda, et al. (2017) Sci. Rep.,7:41478), or 3) a guide RNA having internal loops (Lehmann, et al. (1999) J. Mol. Biol., 291(1): 1-13). As seen in Figure 7, the guide RNAs, including the guide RNA comprising 2 BoxB stem loops, were able to recruit full-length ADAR2 to edit *Mecp2* RNA in transfected HEK cells. These results demonstrate the recruitment of full length ADARs to the target RNA that may include, in addition to target RNA sequences, the presence of sequences that are not normally included in the target RNA.

While certain of the preferred embodiments of the present invention have been described and specifically exemplified above, it is not intended that the invention be limited to such embodiments. Various modifications may be made thereto without departing from the scope and spirit of the present invention, as set forth in the following claims.

## Claims

1. A method for editing a target sequence of an endogenous RNA in a cell, preferably wherein said endogenous RNA is within the nucleus of said cell, said method comprising delivering to the cell:
a) a nucleic acid molecule encoding a fusion protein comprising an RNA editing enzyme, preferably an Adenosine Deaminase Acting on RNA (ADAR) enzyme, linked to an RNA binding domain; and
b) a nucleic acid molecule encoding a guide RNA,
wherein said fusion protein comprises a nuclear localization signal, wherein said guide RNA comprises a sequence specifically recognized by said RNA binding domain, and wherein said guide RNA specifically hybridizes with the target sequence in said endogenous RNA and comprises a mismatch at a nucleotide to be edited.

2. A method according to claim 1, wherein:
- said RNA binding domain is the λN peptide or a variant thereof;
- said sequence specifically recognized by said RNA binding domain is a BoxB sequence; and/or
- said nuclear localization signal is the SV40 Large T-antigen nuclear localization signal or a variant thereof.

3. A method according to claim 1 or 2, wherein said endogenous RNA is an RNA which is expressed in a central nervous system cell, preferably wherein said endogenous RNA is methyl CpG binding protein 2 (MECP2) RNA.

4. A method according to any one of claims 1-3, wherein said guide RNA further comprises one or more mismatches upstream or downstream of the nucleotide to be edited.

5. A method according to any one of claims 1-4, wherein the nucleic acid molecules of a) and b) are contained within a single vector, preferably wherein said vector is a viral vector, more preferably wherein said viral vector is an adeno-associated virus (AAV).

6. A method according to any one of claims 1-5, wherein the guide RNA is able to correct a C to T nonsense mutation by deamination of an A in the 3' position.

7. A combination of
a) a nucleic acid molecule encoding a fusion protein comprising an RNA editing enzyme, preferably an Adenosine Deaminase Acting on RNA (ADAR) enzyme, linked to an RNA binding domain, and
b) a nucleic acid molecule encoding a guide RNA,
for use in a method of treating, inhibiting, and/or preventing a genetic disease of the central nervous system in a subject, said use comprising editing a target sequence of an endogenous RNA in a cell, preferably wherein said endogenous RNA is within the nucleus of said cell,
wherein said fusion protein comprises a nuclear localization signal,
wherein said guide RNA comprises a sequence specifically recognized by said RNA binding domain, and wherein said guide RNA specifically hybridizes with the target sequence in said endogenous RNA and comprises a mismatch at a nucleotide to be edited.

8. A combination for use according to claim 7, wherein:
- said RNA binding domain is the λN peptide or a variant thereof;
- said sequence specifically recognized by said RNA binding domain is a BoxB sequence; and/or
- said nuclear localization signal is the SV40 Large T-antigen nuclear localization signal or a variant thereof.

9. A combination for use according to claim 7 or 8, wherein said endogenous RNA is an RNA which is expressed in a central nervous system cell, preferably wherein said endogenous RNA is methyl CpG binding protein 2 (MECP2) RNA.

10. A combination for use according to any one of claims 7-9, wherein said guide RNA further comprises one or more mismatches upstream or downstream of the nucleotide to be edited.

11. A combination for use according to any one of claims 7-10, wherein the nucleic acid molecules of a) and b) are contained within a single vector, preferably wherein said vector is a viral vector, more preferably wherein said viral vector is an adeno-associated virus (AAV).

12. A combination for use according to any one of claims 7-11, wherein the guide RNA is able to correct a C to T nonsense mutation by deamination of an A in the 3' position.

13. A combination for use according to any one of claims 7-12, wherein the genetic disease of the central nervous system is neonatal encephalopathy, microcephaly, X-linked intellectual disability, PPM-X syndrome, bipolar disorder, parkinsonism, increased muscle tone, exaggerated reflexes, and macroorchidism, or combinations thereof.

14. A combination for use according to any one of claims 7-12, wherein the genetic disease of the central nervous system is Rett syndrome.

15. A vector comprising a nucleic acid molecule encoding a fusion protein comprising an RNA editing enzyme, preferably an Adenosine Deaminase Acting on RNA (ADAR) enzyme linked to an RNA binding domain, wherein said fusion protein comprises a nuclear localization signal.

16. A vector comprising a nucleic acid molecule encoding a guide RNA wherein said guide RNA comprises a sequence specifically recognized by an RNA binding domain.

17. A vector comprising:
a) a nucleic acid molecule encoding a fusion protein comprising an RNA editing enzyme, preferably an Adenosine Deaminase Acting on RNA (ADAR) enzyme, linked to an RNA binding domain; and
b) a nucleic acid molecule encoding a guide RNA,
wherein said fusion protein comprises a nuclear localization signal, wherein said guide RNA comprises a sequence specifically recognized by said RNA binding domain, and wherein said guide RNA specifically hybridizes with the target sequence in said endogenous RNA and comprises a mismatch at a nucleotide to be edited.
